(19) Europäisches Patentamt
European Patent Office
Office européen des brevets

(11) Publication number: **0 338 010 B1**

(12) # EUROPEAN PATENT SPECIFICATION

(45) Date of publication of patent specification: **05.02.92 Bulletin 92/06**

(51) Int. Cl.$^5$: **A61K 31/44,** A61K 31/505, A61K 31/435, A61K 31/47, A61K 31/495, A61K 31/50

(21) Application number: **88900504.7**

(22) Date of filing: **04.12.87**

(86) International application number: **PCT/US87/03112**

(87) International publication number: **WO 88/04172 16.06.88 Gazette 88/13**

(54) **PHARMACEUTICAL COMPOSITIONS FOR TREATMENT OF HYPERPROLIFERATE SKIN DISEASE.**

(30) Priority: **05.12.86 US 938196**
**05.12.86 US 938217**
**18.02.87 US 15829**

(43) Date of publication of application: **25.10.89 Bulletin 89/43**

(45) Publication of the grant of the patent: **05.02.92 Bulletin 92/06**

(84) Designated Contracting States: **AT BE CH DE FR GB IT LI LU NL SE**

(56) References cited:
**EP-A- 0 127 135**
**EP-A- 0 144 996**
**WO-A-86/07537**

(56) References cited:
**WO-A-87/00752**
**The Merck Manual of Diagnosis and Therapy, 14th Edition, 1982 Merck Sharp and Dohme Research Laboratories, Rahway, N.J., US., pages 2026-2034 and 2053-2057.**

(73) Proprietor: **SCHERING CORPORATION**
**2000 Galloping Hill Road**
**Kenilworth New Jersey 07033 (US)**

(72) Inventor: **BLYTHIN, David, J.**
**487 Mountain Avenue**
**North Caldwell, NJ 07066 (US)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1 (DE)**

EP 0 338 010 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to the use of certain azanaphthalenesof the formulae Ia, Ib and Ic defined below in the preparation for pharmaceutical compositions useful in treating hyperproliferative skin disease in mammals.

The compounds of the formulae Ia, Ib and Ic are known compounds: Compounds Ia and methods for their preparation are disclosed in the published European patent application No. 84114974.3; publication No. 0144996. Compounds Ib and methods for their preparation are disclosed in PCT application No. U.S. 86/01518 which has the Publication No. WO 87/00752. Compounds Ic and methods for their preparation are disclosed in the published European patent application No. 84105923.1 which has the publication No. 0127135. All compounds are known to have anti-allergic, anti-inflammatory and/or cyto-protective effect. Compounds Ia and Ic are also described as having immune response supressing activity in WO86/07537 published on December 31, 1986.

It has now surprisingly been found that the compounds also are useful in treatment of hyperproliferative skin diseases. As used herein, this term means any condition the symptoms of which include accelerated skin cell production manifested as scaling, plaques or papular lesions. Examples of hyperproliferative skin diseases include psoriasis, eczema, dandruff, etc.

The compounds useful in the present invention are azanaphtalene compounds of formulae Ia, Ib or Ic or solvates thereof:

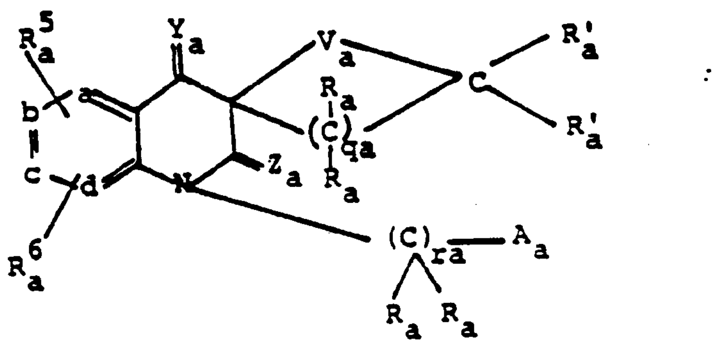

$I_a$

wherein:

two of the ring groups a, b, c and d may be CH or N and the remaining two groups represent CH;

$Y_a$ and $Z_a$ independently represent O or S;

$V_a$ represents 0, $S(O)_{na}$, N-$R^8_a$ or $C(R_a)_2$;

each $R_a$ independently represents hydrogen, alkyl having from 1 to 6 carbon atoms, $CH_2OH$, $COR^7_a$ (wherein $R^7_a$ represents hydrogen or alkyl having from 1 to 6 carbon atoms) or hydroxy, with the proviso that only one hydroxy group can be attached to one carbon atom;

each $R'_a$ independently is as defined for $R_a$ above, except that when $V_a$ represents O, $S(O)_{na}$ or N-$R^8_a$, $R'_a$ may not be hydroxy;

$R^8_a$ is hydrogen, alkyl having from 1 to 6 carbon atoms, carboxylic acyl having from 2 to 7 carbon atoms, alkylsulfonyl having from 1 to 6 carbon atoms, carboalkoxy having from 2 to 7 carbon atoms, $CONH_2$, phenyl or pyridyl of which the last two may be substituted with up to three substituents $Q_a$, whereby each $Q_a$ independently is hydroxy, alkyl having from 1 to 6 carbon atoms, halogen, nitro, alkoxy having from 1 to 6 carbon atoms, trifluoromethyl, cyano, cycloalkyl having from 3 to 7 carbon atoms, alkenyloxy having from 3 to 6 carbon atoms, alkynyloxy having from 3 to 6 carbon atoms, $S(O)_{na}$-$R_a$ {wherein na is as defined above and $R^9_a$ is alkyl having from 1 to 6 carbon atoms}, $NHSO_2R^9_a$ {wherein $R^9_a$ is as defined above}, $NHSO_2CF_3$, $SO_2NH_2$, $COR^{10}_a$ {wherein $R^{10}_a$ is OH, $NH_2$ or $OR^9_a$ (wherein $R^9_a$ is as defined above)}, O-$B_a$-$COR^{10}_a$ {wherein $B_a$ is alkylene having from 1 to 4 carbon atoms and $R^{10}_a$ is as defined above}, or $NHCOR^{11}_a$ {wherein $R^{11}_a$ is hydrogen, alkyl having from 1 to 6 carbon atoms, alkoxy having from 1 to 6 carbon atoms, $COR^{12}_a$ (wherein $R^{12}_a$ is hydroxy or alkoxy having from 1 to 6 carbon atoms) or $NHR^{13}_a$ (wherein $R^{13}_a$ is hydrogen or alkyl having 1 to 6 carbon atoms)};

$R^5_a$ and $R^6_a$ may be the same or different and are hydrogen, alkyl having from 1 to 6 carbon atoms, halogen, nitro, alkoxy having from 1 to 6 carbon atoms, trifluoromethyl, alkylthio having 1 to 6 carbon atoms or cyano;

na is 0, 1 or 2;

ra is 0, 1 or 2;

qa is an integer of from 1 to 5; and

$A_a$ is phenyl, naphthyl, indenyl, indanyl, 2-, 3- or 4-pyridyl, 2-, 4-, 5- or 6-pyrimidyl, 2- or 3-pyrazinyl, 2- or 3-furyl, 2- or 3-thienyl, 2-, 4- or 5-imidazolyl, 2-, 4- or 5-thiazolyl or 2-, 4- or 5-oxazolyl, any of which may be

substituted with up to three substituents $Q_a$ as defined herein above;

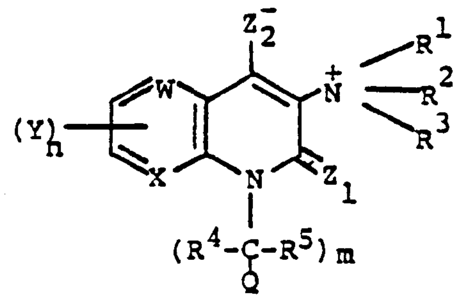

Ib

or pharmaceutical acceptable salts, solvates, or esters thereof, in a combination with a pharmaceutically acceptable carrier, wherein:

W and X may be the same or different and each independently represents -CH= or -N= ;

$Z_1$ and $Z_2$ are the same or different and each independently represents O or S ;

$R^1$, $R^2$, $R^3$, $R^4$ and $R^5$ are the same or different and each may be independently selected from the group consisting of H, alkyl having from 1 to 12 carbon atoms, alkenyl having from 3 to 8 carbon atoms, alkynyl having from 3 to 8 carbon atoms, alkoxyalkyl having from 1 to 6 carbon atoms in the alkoxy portion and from 2 to 6 atoms in the alkyl portion thereof, hydroxyalkyl having from 2 to 8 carbon atoms, cycloalkyl having from 3 to 8 carbon atoms, acyloxyalkyl having from 1 to 6 carbon atoms in the acyloxy portion and from 2 to 8 carbon atoms in the alkyl portion thereof, and $-R^6-CO_2R^0$ wherein $R^6$ represents an alkylene group having from 1 to 6 carbon atoms and $R^0$ represents hydrogen or an alkyl group having from 1 to 6 carbon atoms, with the provisos that in $R^1$, $R^2$, or $R^3$ the OH of the hydroxyalkyl group and the acyloxy of the acyloxyalkyl group are not joined to a carbon atom that is attached to the nitrogen atom to which $R^1$, $R^2$, and $R^3$ are attached and that, when $R^1$, $R^2$ and/or $R^3$ are alkenyl or alkynyl, there is at least one carbon-carbon single bond between the nitrogen atom to which $R^1$, $R^2$, and $R^3$ are attached and the carbon-carbon double or triple bond;

in addition, one of $R^1$, $R^2$ or $R^3$ can,be phenyl which may be substituted with one to three substituents Y as defined below;

in further addition, two of $R^1$, $R^2$ and $R^3$ can be joined together to represent a ring which can contain from 2 to 8 carbon atoms, said ring optionally containing an -O-, -S- and/or $-NR^4-$ group in the ring (wherein $R^4$ is as defined above) and/or optionally containing a carbon-carbon double bond, and said ring optionally being substituted with one to three additional substituents $R^7$ which substituents may be the same or different and are each independently selected from OH with the proviso that OH is not on a carbon already joined to a hetero atom, acyloxy having from 1 to 6 carbon atoms, hydroxyalkyl having from 1 to 8 carbon atoms, alkoxyalkyl having from 1 to 6 carbon atoms in each of the alkoxy and alkyl portions thereof, alkyl having from 1 to 6 carbon atoms, alkenyl having from 3 to 8 carbon atoms, alkynyl having from 3 to 8 carbon atoms, or any two $R^7$ substituent groups may represent a hydrocarbon ring having from 4 to 8 total carbon atoms; and

in still further addition, all three of $R^1$, $R^2$ and $R^3$ together with the nitrogen atom to which they are attached can be joined together to represent a quinuclidine or aza-bicyclo[3.2.2]nonane ring, which ring can optionally be substituted by one to three substituent groups $R^7$ as defined above;

m is an integer of from 0 to 3;

n is an integer of from 0 to 2;

Q represents thiazolyl or phenyl optionally substituted with 1 to 3 substituents Y as defined below; and

each Y substituent is independently selected from the group consisting of hydroxy, alkyl having from 1 to 6 carbon atoms, halogen, $NO_2$, alkoxy having from 1 to 6 carbon atoms, trifluoromethyl, cyano, cycloalkyl having from 3 to 7 carbon atoms, alkenyloxy having from 3 to 6 carbon atoms, alkynyloxy having from 3 to 6 carbon atoms, hydroxyalkyl having from 1 to 6 carbon atoms, $-S(O)_n-R^8$ (wherein $R^8$ represents alkyl having from 1 to 6 carbon atoms and n is as defined above), $-SO_2NH_2$, $-CO-R^9$ (wherein $R^9$ represents OH, $-NH-R^8$ or $-O-R^8$, where $R^8$ is as defined above), $-O-B-COR^9$ (wherein B represents an alkylene group having from 1 to 4 carbon atoms and $R^9$ is as defined above), $-NH_2$, -NHCHO, $-NH-CO-R^9$ (wherein $R^9$ is as defined above, with the proviso that it is not hydroxy), $-NH-COCF_3$, $-NH-SO_2R^8$ (wherein $R^8$ is as defined above), and $-NHSO_2CF_3$;

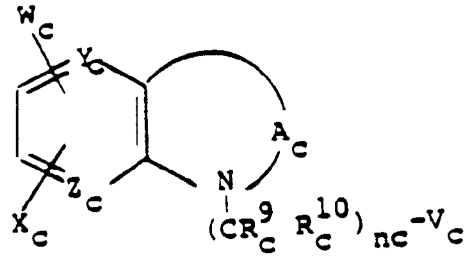

$I_c$

wherein:

$A_c$ is

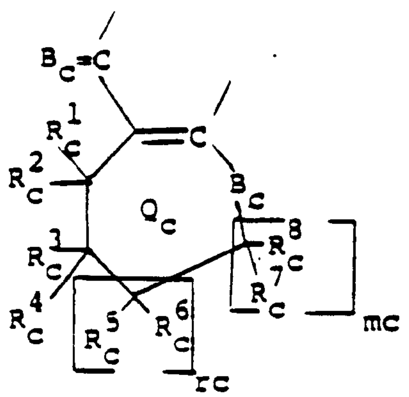

$A_c'$

or

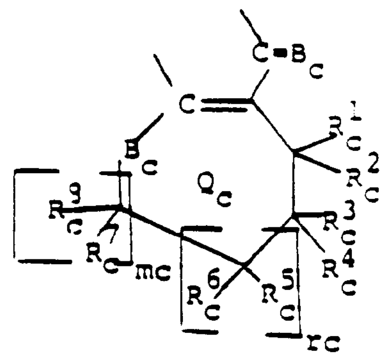

$A_c''$

$B_c$ is independently oxygen or sulfur;

$R^1_c$-$R^8_c$ may be the same or different and are hydrogen or alkyl having from 1 to 6 carbon atoms or two adjacent $R^1_c$-$R^8_c$ substituents may be combined to form an additional carbon to carbon bond;

rc and mc may be the same or different and are 0 or one;

the ring labeled $Q_c$ may optionally contain up to two additional double bonds;

nc is 0, 1 or 2;

$W_c$ and $X_c$ may be the same or different and are hydrogen, hydroxy, alkyl having from 1 to 6 carbon atoms, halogen, nitro, alkoxy having from 1 to 6 carbon atoms, tri-fluoromethyl, cyano, cycloalkyl having from 3 to 7 carbon atoms, alkenyloxy having from 3 to 6 carbon atoms, alkynyloxy having from 3 to 6 carbon atoms, $S(O)_{pc}$-$R^{11}_c$ {wherein pc is 0, 1 or 2 and $R^{11}_c$ is alkyl having from 1 to 6 carbon atoms}, $NHSO_2R^{11}_c$ {wherein $R^{11}_c$ is as defined above}, $NHSO_2CF_3$, $NHCOCF_3$, $SO_2NH_2$, $COR^{12}_c$ {wherein $R^{12}_c$ is OH, $NH_2$ or $OR^{11}_c$ (wherein $R^{11}_c$ is as defined above)}, O-$D_c$-$COR^{12}_c$ {wherein $D_c$ is alkylene having from 1 to 4 carbon atoms and $R^{12}_c$ is as defined above}, or $NHCOR^{13}_c$ {wherein $R^{13}_c$ is hydrogen, alkyl having from 1 to 6 carbon atoms, alkoxy having from 1 to 6 carbon atoms, $COR^{14}_c$ (wherein $R^{14}_c$ is hydroxy or alkoxy having from 1 to 6 carbon atoms) or $NHR^{15}_c$ (wherein $R^{15}_c$ is hydrogen or alkyl having from 1 to 6 carbon atoms)}, or phenoxy {wherein the benzene ring may be substituted with any of the other substituents $W_c$ and $X_c$};

$Y_c$ and $Z_c$ may be the same or different and are CH or N;

$V_c$ is phenyl, naphthyl, indenyl, indanyl, 2-, 3- or 4-pyridyl, 2-, 3- or 5-pyrimidinyl, 2- or 3-thienyl, 2- or 3-furyl or 2-, or 4- or 5-thiazolyl, any of which may be substituted with $W_c$ and $X_c$ as defined above; and $R^9_c$ and $R^{10}_c$ are independently hydrogen or alkyl having from 1 to 6 carbon atoms.

The compounds of formulae Ia, Ib and Ic can exist in unsolvated as well as solvated forms, including hydrated forms. In general, the solvated forms, with pharmaceutically acceptable solvents such as water or ethanol are equivalent to the unsolvated forms for purposes of the invention.

Certain compounds of the invention may exist in isomeric forms. The invention contemplates all such isomers both in pure form and in admixture, including racemic mixtures.

A preferred subgenus of compounds of formula $I_a$ is that wherein $Y_a$ and $Z_a$ are both oxygen.

Preferably, a, b and c represent CH and d is either CH or N. A further preferred feature is that ra is zero, i.e. the group $A_a$ is directly attached to the ring-nitrogen atom. qa is preferably 2, 3 or 4, $V_a$ most preferably is $CH_2$ or O and $R^5_a$ and $R^6_a$ are both hydrogen.

Particularly preferred are compounds of the structural formula $II_a$:

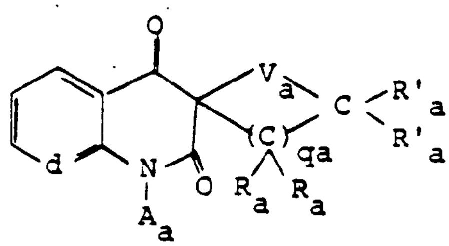

$II_a$

wherein d is CH or N, qa is 2, 3 or 4 and $A_a$, $V_a$, $R_a$ and $R'_a$ are as defined above.

When utilized herein and in the appended claims the below listed terms, unless specified otherwise, are defined as follows:

halogen - comprises fluorine, chlorine, bromine and iodine;

alkyl and alkoxy - comprises straight and branched carbon chains containing from 1 to 6 carbon atoms;

alkenyloxy - comprises straight and branched carbon chains containing from 3 to 6 carbon atoms and comprising a carbon to carbon double bond; and

alkynyloxy - comprises straight and branched carbon chains containing from 3 to 6 carbon atoms and comprising a carbon to carbon triple bond.

The compounds include a

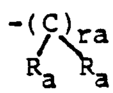 and a 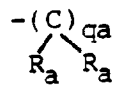

- substituent wherein the $R_a$ groups may vary independently. Thus, for example, when ra or qa equals 2 the following patterns of substitution (wherein hydrogen and $CH_3$ are used to represent any substituents $R_a$) are contemplated: $-C(CH_3)_2CH_2-$, $-CH_2C(CH_3)_2-$, $-CH_2CH(CH_3)-$, $-CH(CH_3)CH_2-$, $-(C(CH_3)H)_2-$ and the like. In addition when ra or qa equals 2, substituents such as $-C(CH_3)_2CH(C_2H_5)-$, $-CH(CH_3)CH(C_2H_5)$ are also contemplated.

It is obvious to one of ordinary skill in the art that due to problems of stability there are limitations involving the $R_a$ and $R'_a$ groups. One limitation is that neither $R_a$ can be a hydroxy group attached to the carbon alpha to the ring nitrogen atom. Another limitation is that the $R_a$ and $R'_a$ groups cannot both be hydroxy groups attached to the same carbon atoms.

Certain compounds may exist in isomeric forms. The invention contemplates all such isomers both in pure form and in admixture, including racemic mixtures. In the structural formulas $I_a$ and $II_a$ herein, when $V_a$ represents a hetero atom in the spiro ring, $V_a$ is attached directly to the spiro carbon atom, i.e., the carbon atom identified as number 3 in structural formula $I_a$.

The preferred compounds of formula $I_a$ summerized as follows:

compounds of formula $I_a$ wherein $Y_a$ and $Z_a$ are both oxygen and/or

ra is zero and/or

$R^5_a$ and $R^6_a$ are both hydrogen and/or

qa is 2, 3 or 4 and/or

$R_a$ and $R'_a$ independently are hydrogen, methyl, ethyl, propyl, isopropyl, n-butyl or iso-butyl and/or

$A_a$ is phenyl or phenyl substituted with one or two substituents $Q_a$ and/or

$V_a$ is 0, $S(O)_{na}$ or $N-R^8_a$ preferably O;

wherein all of a, b, c, and d are CH or

d is N and a, b and c are CH.

Representative compounds of formula $I_a$ are exemplified below in Table I:

TABLE I

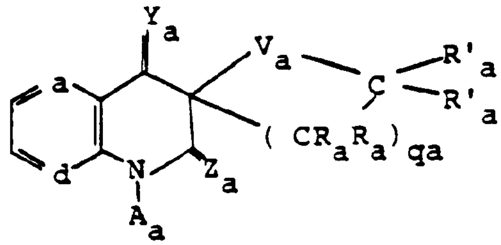

$III_a$

| Compound No. | d | a | $A_a$ | $Y_a$ | $Z_a$ | $V_a$ | $-C(R'_a)_2(CR_aR_a)_{ma}-$ | m.p. (°C) |
|---|---|---|---|---|---|---|---|---|
| 1 | N | CH | phenyl | O | O | $CH_2$ | trimethylene | 174-178 |
| 2 | N | CH | 3-hydroxy phenyl | O | O | $CH_2$ | trimethylene | 218-220 |
| 3 | N | CH | 3-methoxy phenyl | O | O | $CH_2$ | trimethylene | 159-160.5 |
| 4 | CH | CH | phenyl | O | O | $CH_2$ | trimethylene | 168-168 |
| 5 | N | CH | 3,4-chloro phenyl | O | O | $CH_2$ | trimethylene | 143-145.5 |
| 6 | N | CH | 4-chloro phenyl | O | O | $CH_2$ | trimethylene | 168.5-172 |
| 7 | N | CH | 4-methyl phenyl | O | O | $CH_2$ | trimethylene | 177-178.5 |
| 8 | N | CH | 3-chloro phenyl | O | O | $CH_2$ | trimethylene | 165-167 |
| 9 | N | CH | 3-chloro phenyl | S | O | $CH_2$ | trimethylene | 168-170 |
| 10 | N | CH | phenyl | S | O | $CH_2$ | trimethylene | 188-189.5 |
| 11 | N | CH | 3-ethyloxalyl-amino phenyl | O | O | $CH_2$ | trimethylene | 158-160 |
| 12 | N | CH | 3-formylamino-phenyl | O | O | $CH_2$ | trimethylene | 222-224 |
| 13 | N | CH | 3-aminophenyl | O | O | $CH_2$ | trimethylene | 200-202 |
| 14 | N | CH | 3-(ethyloxy-carbonyl-methoxy)phenyl | O | O | $CH_2$ | trimethylene | 103-105 |
| 15 | N | CH | phenyl | O | O | O | trimethylene | 241.5-243 (1/3 hydrate) |
| 16 | N | CH | phenyl | O | O | O | ethylene | 233-235.5 |
| 18 | N | CH | phenyl | O | O | S | trimethylene | |
| 19 | N | CH | 3,4-dichloro phenyl | O | O | O | tetramethylene | |
| 20 | N | CH | 3-chloro-phenyl | O | O | O | tetramethylene | 158.5-160 |
| 21 | N | CH | 4-chloro-phenyl | O | O | O | tetramethylene | 229-231.5 (hemihydrate) |
| 22 | N | CH | 3-methoxy-phenyl | O | O | O | tetramethylene | 181-183 |
| 23 | N | CH | 4-methoxy phenyl | O | O | O | tetramethylene | |

A preferred subgenus of compounds Ib is represented by those compounds in which at least one of W and X is N. More preferably, W is CH and X is N. Moreover, at least one of $Z_1$ and $Z_2$ is preferably O and m and n are preferably 0.

An additional preferred subgenus of compounds is represented by the structural formula IIb.

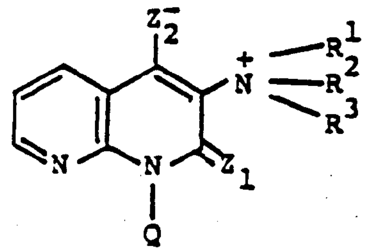

(IIb)

wherein $R^1$, $R^2$, $R^3$, Q, $Z_1$ and $Z_2$ are as defined above. Preferably, at least one of $Z_1$ and $Z_2$ is O. In addition, Q is preferably phenyl, which may be optionally substituted with one to three Y groups, more preferably, one or two Y groups.

The compounds, when in their zwitterionic form, have good solubility in physiological fluids, such as blood, plasma, saliva, etc., and in general in polar solvents, such as water and ethanol, which can be used in compositions for delivering the compounds to patients. This characteristic is advantageous in that the compounds are expected to be more easily absorbed gastrointestinally and therefore provide good activity when administered orally. Of course compounds in other forms, such as esters, also have therapeutic activity. propynyloxy, hydroxyethyl, methylthio, methylsulfonyl, carboxy, acetoxy, N-methylaminocarbonyl, acetoxymethoxy, acetamido or methylsulfonamido.

Exemplary compounds within the scope of formula IIb are:

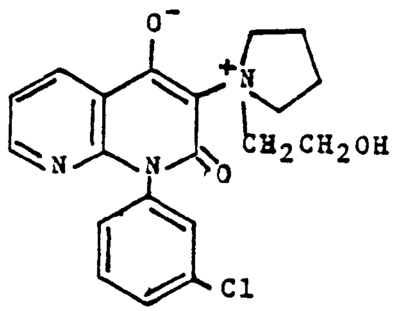

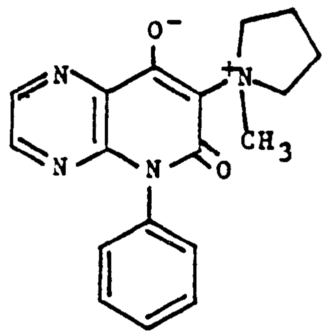

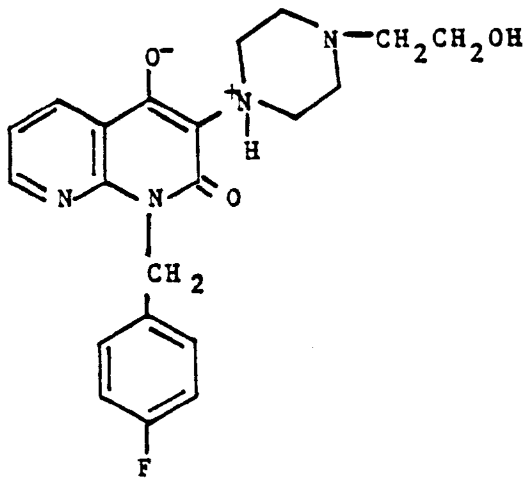

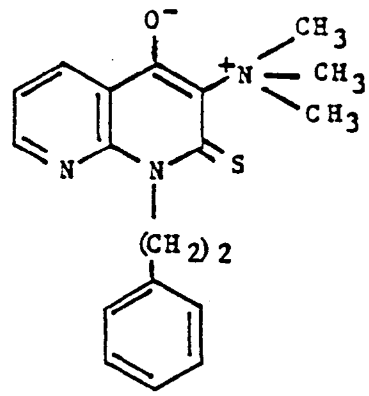

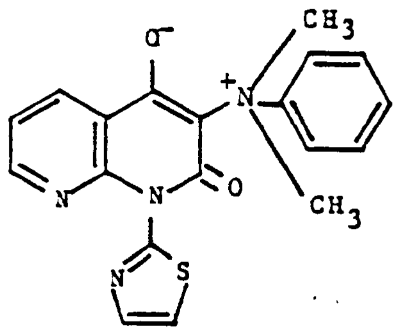

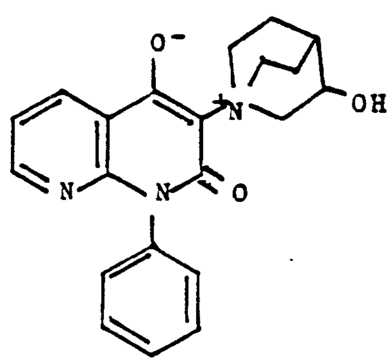

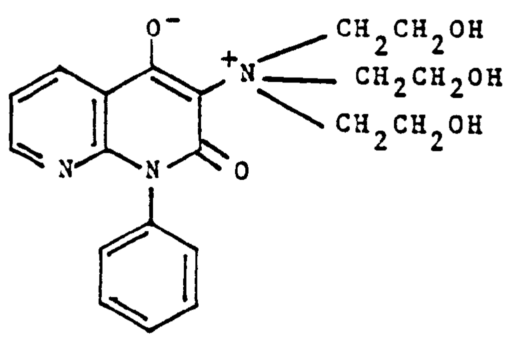

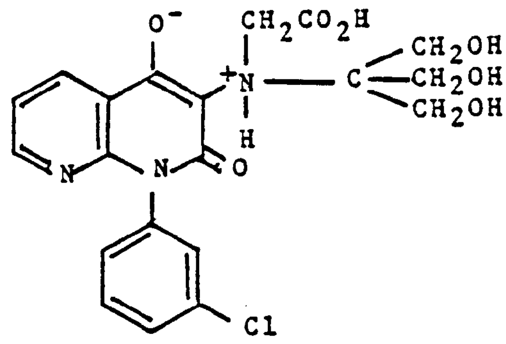

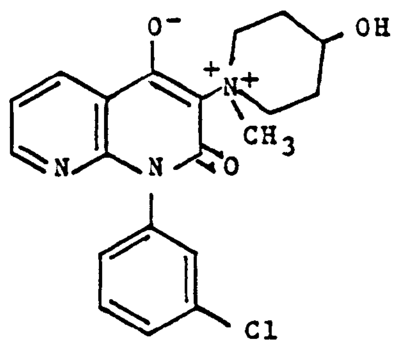

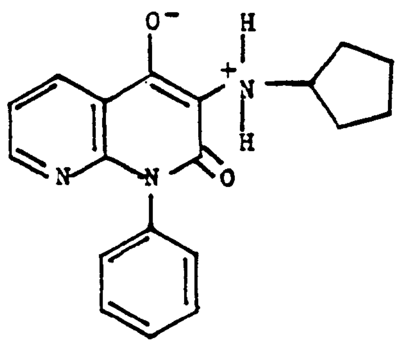

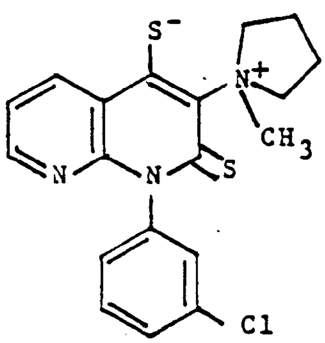

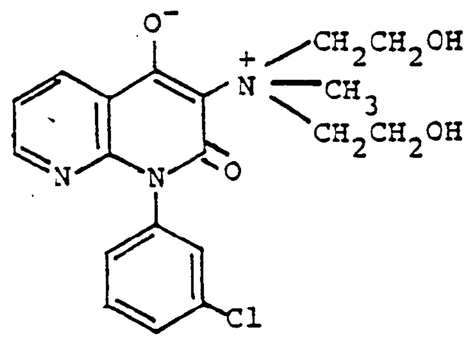

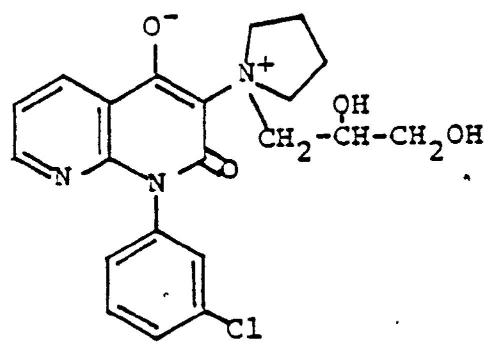

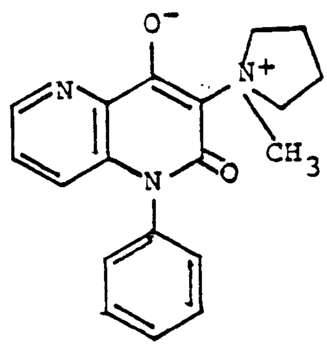

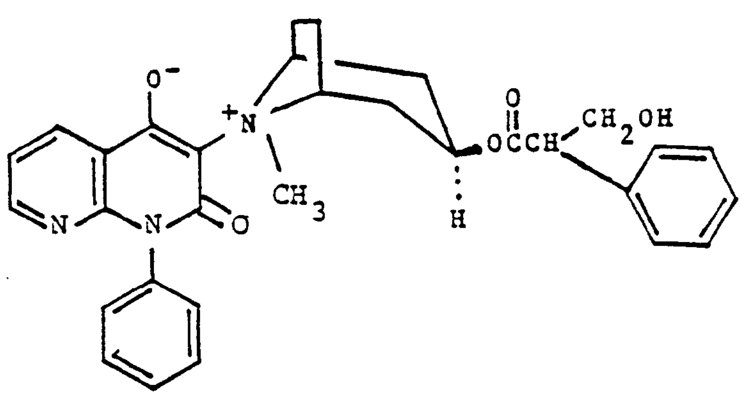

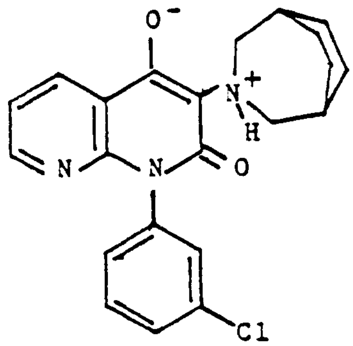

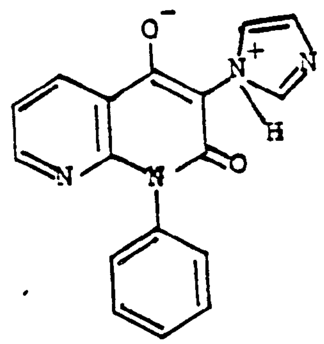

The compounds are zwitterionic or inner salts, i.e., they are both positively and negatively charged. However, pharmaceutically acceptable salts of such compounds are also included, i.e., pharmaceutically acceptable acid addition or basic salts. Examples of suitable acid addition salts include the chloride (from hydrochloric acid), methyl sulfate (from methyl sulfuric acid) sulfate (from sulfuric acid) and bromide. Basic salts can be formed when at least one of $R^1$, $R^2$ and $R^3$ is H. Examples of suitable basic salts include sodium, potassium or calcium salts (from their corresponding hydroxides). In addition, esters of the zwitterionic compounds are within the scope of this invention. Such esters may be formed, e.g., by reacting the zwitterionic compounds with an acyl halide in solvent, such as methylene chloride and base, such as triethylamine.

The compounds of formula Ib can exist in unsolvated as well as solvated forms, including hydrated forms, e.g., a hemihydrate. Hydrates and other solvates are formed by contacting the unsolvated form with the solvent. In general, the solvated forms, with pharmaceutically acceptable solvents such as water or ethanol are equivalent to the unsolvated forms for purposes of the invention.

Certain compounds may exist in isomeric and tautomeric forms. The invention includes all such isomers and tautomers - the isomers both in pure form and in admixture, including racemic mixtures.

Subgenera of compounds of formula $I_c$ are

compounds wherein $B_c$ is oxygen;

compounds wherein $B_c$ is oxygen and $A_c$ is $A'_c$;

compounds wherein nc is zero and/or $Y_c$ is CH;

typical subgeneric groups of compounds of formula $I_c$ are

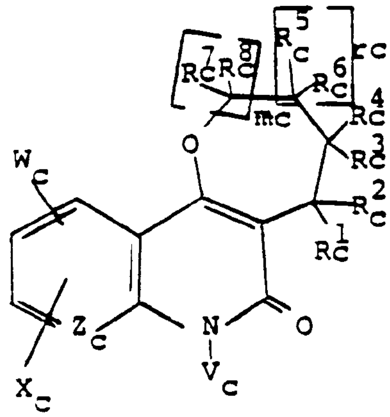

1

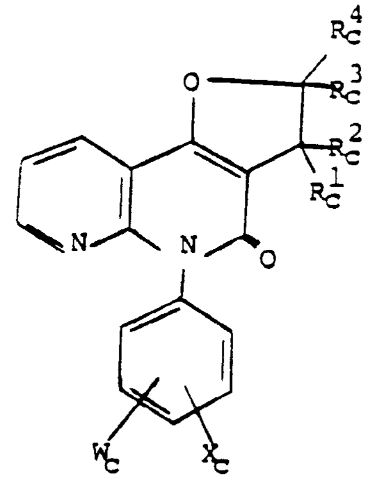

2

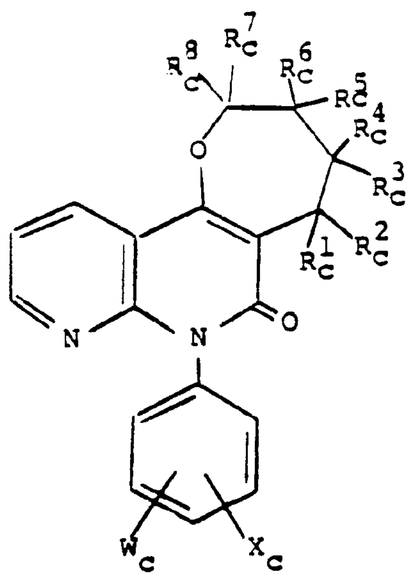

3

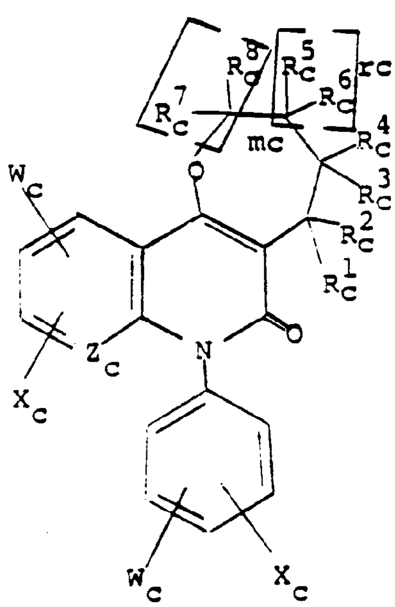

4

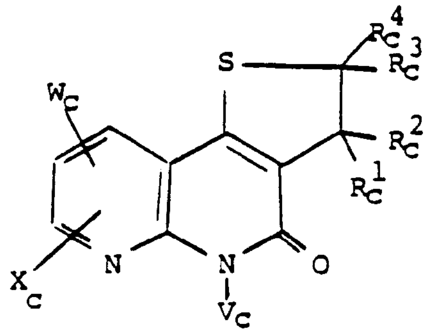

5

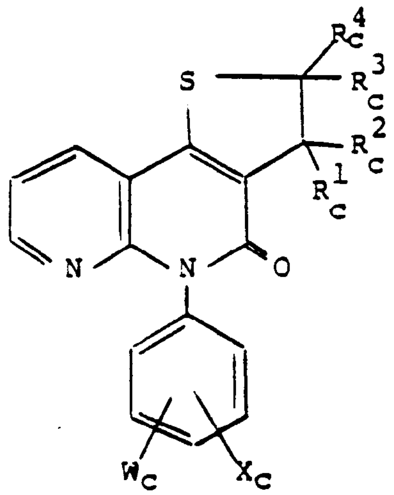

6

wherein $W_c$, $V_c$, $X_c$, $Z_c$, $R^1{}_c$-$R^8{}_c$, rc and mc are as defined herein;

compounds of formula 2

wherein $W_c$, $X_c$, $R^1{}_c$, $R^2{}_c$, $R^3{}_c$, and $R^4{}_c$ are as defined above, wherein preferably $R^1{}_c$, $R^2{}_c$, $R^3{}_c$ and $R^4{}_c$ are Hydrogen or methyl;

in particular compounds of formula 1

wherein $W_c$, $X_c$, $Z_c$, $V_c$, $R^1{}_c$ to $R^8{}_c$, rc and mc are as defined above

wherein preferably

$Z_c$ is N and/or

rc and mc are both zero or one or

the sum of rc and mc is one and/or

$B_c$ is oxygen and/or

$V_c$ is of formula 7;

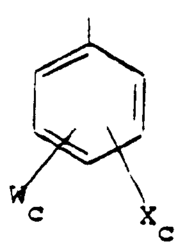

7

compounds of formula 3

wherein $W_c$, $X_c$ and $R^1{}_c$-$R^8{}_c$ are as defined above, wherein preferably

$R^1{}_c$-$R^8{}_c$ are hydrogen or methyl, especially hyrogen, or one of $R^1{}_c$-$R^8{}_c$ is methyl and the rest are hydrogen and/or

$W_c$ is 3-chloro and $X_c$ is hydrogen, chlorine or fluorine or
$W_c$ is 3-methoxy and $X_c$ is hydrogen or fluorine or
$W_c$ and $X_c$ are both hydrogen;

compounds of formula 5

wherein $W_c$, $X_c$, $V_c$, $R^1_c$, $R^2_c$, $R^3_c$ and $R^4_c$ are as defined above;

compounds of formula 6

wherein $W_c$, $X_c$, $R^1_c$, $R^2_c$, $R^3_c$ and $R^4_c$ are as defined above, wherein preferably
$W_c$ is 3-chloro and $X_c$ is hydrogen, chlorine or fluorine or
$W_c$ is 3-methoxy and $X_c$ is hydrogen or fluorine or $W_c$ and $X_c$ are both hydrogen.

Representative compounds of formula $I_c$ are exemplified by the following compounds:

3,5-dihydro-5-phenyl-furo[3,2-c][1,8]naphthyridin-4[2H]-one;
3,5-dihydro-5-phenyl-thieno[3,2-c][1,8]-naphthyridin-4[2H]-one;
6-phenyl-2,3,4,6-tetrahydro-pyrano[3,2-c][1,8]-naphthyridin-5-one;
2-methyl-3,5-dihydro-5-phenyl-furo[3,2-c][1,8]-naphthyridin-4[2H]-one;
3,9-dihydro-9-phenyl-furo[2,3-b][1,8]naphthyridin-4[2H]-one;
3,9-dihydro-9-(p-methylphenyl)-furo[2,3-b][1,8]naphthyridin-4[2H]-one;
3,9-dihydro-2-methyl-9-phenyl-furo[2,3-b][1,8]naphthyridin-4[2H]-one;
3,5-dihydro-5-(p-methylphenyl)-furo[3,2-c](1,8]naphthyridin-4[2H]-one; .
3,5-dihydro-5-(p-fluorophenyl)-furo[3,2-c][1,8]naphthyridin-4[2H]-one;
3,5-dihydro-5-(m-methoxyphenyl)-furo[3,2-c][1,8]naphthyridin-4[2H]-one;
3,5-dihydro-5-(m-methylthiophenyl)-furo[3,2-c][1,8]-naphthyridin-4[2H]-one;
3,9-dihydro-9-(p-fluorophenyl)-furo[2,3-b][1,8]naphthyridin-4[2H]-one;
3,9-dihydro-9-(m-methoxyphenyl)-furo[2,3-b][1,8]naphthyridin-4[2H]-one;
3,9-dihydro-9(m-methylthiophenyl)-furo[3,2-c][1,8]-naphthyridin-4[2H]-one;
3,5-dihydro-5-(3,4-dichlorophenyl)-furo[3,2-c][1,8]-naphthyridin-4[2H]-one;
3,5-dihydro-5-(3,4-dichlorophenyl)-2-methyl-furo[3,2-c][1,8]-naphthyridin-4[2H]-one;
3,5-dihydro-5-(4-chlorophenyl)-furo[3,2-c][1,8]-naphthyridin-4[2H]-one;
3,5-dihydro-5-(3-chlorophenyl)-furo[3,2-c][1,8]-naphthyridin-4[2H]-one;
3,5-dihydro-5-(3-chlorophenyl)-2-methyl-furo[3,2-c][1,8]-naphthyridin-4[2H]-one;
3,5-dihydro-5-(4-fluorophenyl)-2-methyl-furo[3,2-c][1,8]-naphthyridin-4[2H]-one;
3,5-dihydro-5-(3-methoxyphenyl)-2-methyl-furo [3,2-c][1,8]-naphthyridin-4[2H]-one;
3,5-dihydro-5-(3,5-dichlorophenyl)-furo[3,2-c][1,8]-naphthyridin-4[2H]-one;
3,5-dihydro-5-(3,5-dichlorophenyl)-2-methyl-furo[3,2-c][1,8]-naphthyridin-4[2H]-one;
3,5-dihydro-5-phenyl-2,2-dimethyl-furo[3,2-c][1,8]-naphthyridin-4[2H]-one;
3,5-dihydro-5-(3-methylsulfonylaminophenyl)-2-methyl-furo[3,2-c][1,8]-naphthyridin-4[2H]-one;
3,5-dihydro-5-(3-methylsulfonylaminophenyl)-furo[3,2-c][1,8]-naphthyridin-4[2H]-one;
3,9-dihydro-9-(3,4-dichlorophenyl)-furo[2,3-b][1,8]-naphthyridin-4[2H]-one;
3,9-dihydro-9-(4-chlorophenyl)-furo[2,3-b][1,8]-naphthyridin-4[2H]-one;
3,9-dihydro-9-(3-chlorophenyl)-furo[2,3-b][1,8]-naphthyridin-4[2H]-one;
3,9-dihydro-9-(3-chlorophenyl)-2-methyl-furo[2,3-b][1,8]-naphthyridin-4[2H]-one;
3,9-dihydro-9-(4-fluorophenyl)-2-methyl-furo[2,3-b][1,8]-naphthyridin-4[2H]-one;
3,9-dihydro-9-(3-methoxyphenyl)-2-methyl-furo[2,3-b][1,8]-naphthyridin-4[2H]-one;
3,9-dihydro-9-(3,5-dichlorophenyl)-furo[2,3-b][1,8]-naphthyridin-4[2H]-one;
3,9-dihydro-9-(3,5-dichlorophenyl)-2-methyl-furo[2,3-b][1,8]-naphthyridin-4[2H]-one;
3,9-dihydro-9-(3-methylsulfonylaminophenyl)-2-methyl-furo[2,3-b][1,8]-naphthyridin-4[2H]-one;
6-(4-chlorophenyl)-2,3,4,6-tetrahydro-5H-pyrano[3,2-c][1,8]naphthyridin-5-one;
6-(3,4-dichlorophenyl)-2,3,4,6-tetrahydro-5H-pyrano[3,2-c][1,8]naphthyridin-5-one;
6-(4-methoxyphenyl)-2,3,4,6-tetrahydro-5H-pyrano[3,2-c][1,8]naphthyridin-5-one;
6-(4-methylphenyl)-2,3,4,6-tetrahydro-5H-pyrano [3,2-c][1,8]naphthylidin-5-one;
10-(3,4-dichlorophenyl)-2,3,4,10-tetrahydro-5H-pyrano[2,3-b][1,8]naphthyridin-5-one;
10-(4-methoxyphenyl)-2,3,4,10-tetrahydro-5H-pyrano[2,3-b][1,8]naphthyridin-5-one;
10-(4-chlorophenyl)-2,3,4,10-tetrahydro-5H-pyrano[2,3-b][1,8]naphthyridin-5-one;
10-(4-methylphenyl)-2,3,4,10-tetrahydro-5H-pyrano[2,3-b][1,8]naphthyridin-5-one;
10-phenyl-2,3,4,10-tetrahydro-5H-pyrano-[2,3-b][1,8]naphthyridin-5-one;
7-phenyl-3,4,5,7-tetrahydro-oxepino(3,2-c][1,8]naphthyridin-6[2H]-one;
7-(4-chlorophenyl)-3,4,5,7-tetrahydro-oxepino[3,2-c][1,8]-naphthyridin-6[2H]-one;
7-(3-chlorophenyl)-3,4,5,7-tetrahydro-oxepino[3,2-c][1,8]-naphthyridin-6[2H]-one;
7-(3-methoxyphenyl)-3,4,5,7-tetrahydro-oxepino[3,2-c][1,8]-naphthyridin-6[2H]-one; and

7-(3-hydroxyphenyl)-3,4,5,7-tetrahydro-oxepino[3,2-c][1,8]-naphthyridin-6(2H)-one.

Effectiveness of the compounds for the treatment of hyperproliferative skin disease in mammals is demonstrated by the Arachidonic Acid Mouse Ear Test described below. The Arachidonic Acid Mouse Ear Test is a recognized model for assessing the effectiveness of compounds for the treatment of hyperproliferative skin disease.

Arachidonic Acid Mouse Ear Test, Materials and Methods

Charles River, female, CD, (SD) BR mice, 6 weeks old, are caged 8/group and allowed to acclimate 1-3 weeks prior to use.

Arachidonic acid (AA) is dissolved in reagent grade acetone (2 mg/.01 ml) and stored at -20°C for a maximum of 1 week prior to use. Inflammatory reactions are induced by applying 10 μl of AA to both surfaces of one ear (4 mg total).

Test drugs are dissolved in either reagent grade acetone or aqueous ethanol (only if insoluble in acetone) at the same doses selected by Opas et al., Fed. Proc. 43, Abstract 2983, p. 1927 (1984) and Young et al., J. Invest. Dermatol. 82, pp. 367-371 (1984). These doses are employed to ensure maximum responses and to overcome any difference in topical absorption which could occur with any drug applied in an aqueous ethanol vehicle. The test drug is applied 30 minutes prior to challenge with AA.

The severity of the inflammation is measured as a function of increased ear weight. A 6 mm punch biopsy is removed 1 hour after AA challenge and weighed to the nearest 0.1 mg. Mean ± standard error and all possible comparisons are made via Duncan's Multiple Range Statistic.

The results of the above test for a representative selection of compounds of formula Ia, Ib and Ic are given in Table II:

Compound 1

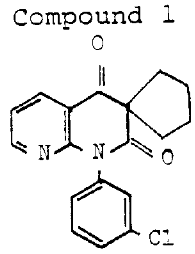

Compound 2

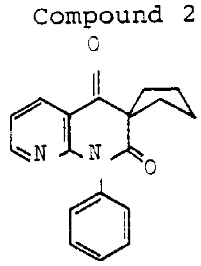

Compound 3

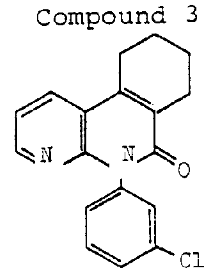

Compound 4

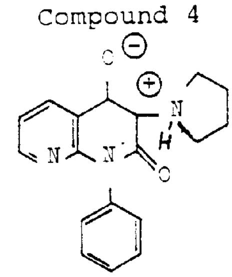

| Compound | Dose (mg/ear) | Tissue Wt (g) | % Inhibition |
|---|---|---|---|
| 1 | 1.0 | 1.33 ± 0.40 | 82 |
| 1 | 0.1 | 2.01 ± 0.69 | 72 |
| 2 | 1.0 | 0.6 ± 0.4 | 90 |
| 2 | 0.5 | 1.0 ± 0.5 | 76 |
| 3 | 1.0 | 0.83 ± 0.34 | 90 |
| 3 | 0.1 | 1.40 ± 0.18 | 83 |
| 4 | 0.5 | 3.01 ± 0.47 | 61 |
| 4 | 0.1 | 4.35 ± 0.01 | 43 |

Effectiveness of the compounds used herein is further demonstrated using, for example, the compound 3,9-dihydro-9-(3-chlorophenyl)-2-methyl-furo[2,3-b][1,8]-naphthyridin-4[2H]-one which exhibits activity at an effective dose ($ED_{50}$) of 0.13 mg which gives 50% inhibition. Based upon the above, the compounds used herein are effective for the treatment of hyperproliferative skin disease. Based upon the test results, the compounds used herein can be administered by any conventional route, e.g., orally, topically, parenterally, etc. in a dosage range of about 0.05 mg/kg to about 50 mg/kg to treat hyperproliferative skin disease.

The dosage to be administered and the route of administration depends upon the particular compound used, the age and general health of the patient and the severity of the inflammatory condition. Thus, the dose ultimately decided upon must be left to the judgent of a trained health-care practitioner.

When administered parenterally, e.g. intravenously, the compounds are administered at a dosage range of about 0.01-10 mg/kg of body weight in single or multiple daily doses.

The compounds can be administered in unit dosage forms such as tablets, capsules, pills, powders, granules, sterile parenteral solutions or suspensions, suppositories and mechanical delivery devices, e.g. transdermal

For preparing pharmaceutical compositions from the compounds described by this invention, inert, pharmaceutically acceptable carriers can be either solid or liquid. Solid form preparations include powders, tablets dispersible granules, capsules, cachets and suppositories. A solid carrier can be one or more substances which may also act as diluents, flavoring agents, solubilizers, lubricants, suspending agents, binders or tablet disintegrating agents; it can also be an encapsulating material. In powders, the carrier is a finely divided solid which is in admixture with the finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable

solid carriers are magnesium carbonate, magnesium stearate, talc, sugar, lactose, pectin, dextrin, starch, gelatin, tragacanth, methylcellulose, sodium carboxymethylcellulose, a low melting wax and cocoa butter. The term "preparation" is intended to include the formulation of the active compound with encapsulating material as carrier providing a capsule in which the active component (with or without other carriers) is surrounded by carrier, which is thus in association with it. Similarly, cachets are included. Tablets, powders, cachets and capsules can be used as solid dosage forms suitable for oral administration.

For the treatment of hyper-proliferative skin disease, the preferred route of administration is topical. In this preferred method, a pharmaceutical formulation comprising the active ingredient together with a non-toxic pharmaceutically acceptable topical carrier, usually in concentrations ranging from about 0.001 percent to about 10 percent, preferably from about 0.1 to about 5 percent, is applied several times daily to the affected skin until the condition has improved. Topical applications may then be continued at less frequent intervals (e.g., once a day) to control the condition.

The compounds may be conveniently applied in a liquid solvent, preferably in a water-miscible liquid carrier made up of hydrophilic liquids having a high solvating action, e.g., a solution which comprises, for example, propylene glycol and polyethylene glycol. Alternatively, the compound may be applied in dry form, such as a powder. Other forms in which the compound may be used topically include creams, lotions, aerosols, dusts and ointments which are prepared by combining the compound with conventional pharmaceutical diluents and carriers commonly used in topical dry, liquid, cream and aerosol formulations.

The ointments and creams may, for example, be formulated with an aqueous oily base with the addition of suitable thickening and/or gelling agents. Such bases may thus, for example, include water and/or oil such as liquid paraffin or vegatable oil, such as peanut oil or castor oil. Thickening agents which may be used according to the nature of the base include soft paraffin, aluminum stearate, cetostearyl alcohol, propylene glycol, polyethylene glycols, woolfat, hydrogenated lanolin or beeswax.

Lotions may be formulated with an aqueous or oily base and will, in general, also include one or more of the following, namely, stabilizing agents, emulsifying agents, dispensing agents, suspending agents, thickening agents, coloring agents or perfumes.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby solidify.

Liquid form preparations include solutions, suspensions and emulsions. As an example may be mentioned water or water-propylene glycol solutions for parenteral injection. Liquid preparations can also be formulated in solution in aqueous polyethylene glycol solution. Aqueous solutions suitable for oral use can be prepared by adding the active component in water and adding suitable colorants, flavors, stabilizing, sweetening, solubilizing and thickening agents as desired. Aqueous suspensions suitable for oral use can be made by dispersing the finely divided active component in water with viscous material, i.e., natural or synthetic gums, resins, methylcellulose, sodium carboxymethylcellulose and other well-known suspending agents.

Also included are solid form preparations which are intended to be converted, shortly before use, to liquid form preparations for either oral or parenteral administration. Such liquid forms include solutions, suspensions and emulsions. These particular solid form preparations are most conveniently provided in unit dose form and as such are used to provide a single liquid dosage unit. Alternatively, sufficient solid may be provided so that after conversion to liquid form, multiple individual liquid doses may be obtained by measuring predetermined volumes of the liquid form preparation as with a syringe, teaspoon or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorants, colorants, stabilizers, buffers, artificial and natural sweeteners, dispersants, thickeners or solubilizing agents. The solvent utilized for preparing the liquid form preparation may be water, isotonic water, ethanol, glycerine or propylene glycol as well as mixtures thereof. Naturally, the solvent utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules and powders in vials or ampoules. The unit dosage form can also be a capsule, cachet or tablet itself or it can be the appropriate number of any of these in packaged form.

The quantity of active compound in a unit dose of preparation may be varied or adjusted from about 0.1 mg to about 500 mg according to the particular application and the potency of the active ingredient. The compositions can, if desired, also contain other therapeutic agents.

The dosages may be varied depending upon the requirements of the patient, the severity of the condition

being treated and the particular compound being employed. Determination of the proper dosage for a particular situation is within the skill of the art. Generally, treatment is initiated with smaller dosages which are less than the optimum dose of the compound. Thereafter, the dosage is increased by small increments until the optimum effect under the circumstances is reached. For convenience, the total daily dosage may be divided and administered in portions during the day if desired.

The following formulations exemplify some of the dosage forms. In each, the active ingredient is a compound represented by structural formulae Ia, Ib or Ic, such as 9-(3-chlorophenyl)-3,9-dihydro-2-methyl-furo [2,3-b] [1,8] naphthyridine-4(2H)-one, and is referred to as "active compound". However, any other compound falling within the scope of formulae Ia, Ib or Ic could be substituted therefore.

FORMULATIONS

### Formulation I: Ointment

| Formula | mg/g |
|---|---|
| Active Compound | 1.0-20.0 |
| Benzyl Alcohol, NF | 10.0 |
| Mineral Oil, USP | 50.0 |
| White Petrolatum, USP to make | 1.0 g |

Procedure

Mix and heat to 65°C, a weighted quantity of white petrolatum, mineral oil, benzyl alcohol, and cool to 50°-55°C. with stirring. Disperse active compound in a portion of the mineral oil and then add to the above mixture with stirring. Cool to room temperature.

### Formulation II: Cream

| Formula | mg/g |
|---|---|
| Active Compound | 1.0-20.0 |
| Stearic Acid, USP | 60.0 |
| Glyceryl Monostearate | 100.0 |
| Propylene Glycol, USP | 50.0 |
| Polyethylene Sorbitan Monopalmitate | 50.0 |
| Sorbitol Solution, USP | 30.0 |
| Benzyl Alcohol, NF | 10.0 |
| Purified Water, USP to make | 1.0 g |

Procedure

Heat the stearic acid, glyceryl monostearate and polyethylene sorbitan monopalmitate to 70°C. In a separate vessel, dissolve sorbital solution, benzyl alcohol, water, and half quantity of propylene glycol and heat to 70°C. Add the aqueous phase to oil phase with high speed lightning stirring. Dissolve the active compound in remaining quantity of propylene glycol and add to the above emulsion when the temperature of emulsion is 37°-40°C. Mix uniformly with stirring and cool to room temperature.

Formulation III: Gel

| Formula | mg./g |
|---|---|
| Active Compound | 1.0-20.0 |
| Propylene Glycol, USP | 300.0 |
| Butylated Hydroxytoluene | 5.0 |
| Carbomer 940 | 5.0 |
| Sodium Hydroxide (added as a 1% w/w solution in proplyene glycol) | 0.7 |
| Polyethylene Glycol 400, USP | 669.3-688. |

Procedure

Prepare a 1% solution of the sodium hydroxide in propylene glycol and hold. Add approximately one-half the remaining propylene glycol, and the polyethylene glycol 400 to a suitable vessel and mix. Dissolve the butylated hydroxytoluene in this mixture. Disperse the carbomer 940 in the above mixture with vigorous agitation. Add the solution of sodium hydroxide with high speed agitation to bring pH up to 7 and recirculation until a thick gel forms. Dissolve the active compound in the remaining propylene glycol and add to the gel slowly as the gel is continuously recirculated.

Formulation IV: Lotion

| Formula | mg/g |
|---|---|
| Active Compound | 1.0-20.0 |
| Carbomer 940 | 3.0 |
| Sodium hydroxide (charged as 4% w/w aqueous solution) | 0.05 |
| Isopropyl Alcohol | 40.00 |
| Purified Water, USP to make | 1.0 g |

Procedure

Prepare a 4% solution of sodium hydroxide in water. Heat the purified water to 60°C. Add carbomer 940 and mix at high speed until dispersed. Cool the above mixture to room temperature and slowly charge sodium hydroxide until uniform. Add 80% of isopropyl alcohol to the above with mixing. Dissolve the active compound in remaining isopropanol. Add this to the mixture with stirring. Adjust pH to 5.0 to 5.5 with sodium hydroxide, if necessary.

Formulation V: Topical Aerosol

| Formula | mg/g |
|---|---|
| Active Compound | 1.0-20.0 |
| Caprylic/Capric Triglyceride | 50.00 |
| Mineral Oil | 20.00 |
| Specially Denatured Alcohol | 150.00 |
| Hydrocarbon Aerosol Propellant q.s. ad. | 1.0g |

Procedure

Add and mix the caprylic/capric triglyceride mineral oil and specially denatured alcohol in a suitable compounding tank. Add the active compound drug and continue mixing until the active compound is dissolved or

dispersed uniformily. Fill the concentrate into cans and then fill the required amount of hydrocarbon aerosol propellant.

## Formulation VI: Tablets

| No. | Ingredient | mg/tablet | mg/tablet |
|---|---|---|---|
| 1. | Active compound | 100 | 500 |
| 2. | Lactose USP | 122 | 113 |
| 3. | Corn Starch, Food Grade, as a 10% paste in Purified Water | 30 | 40 |
| 4. | Corn Starch, Food Grade | 45 | 40 |
| 5. | Magnesium Stearate | 3 | 7 |
| | Total | 300 | 700 |

Method of Manufacture

Mix Item Nos. 1 and 2 in a suitable mixer for 10-15 minutes. Granulate the mixture with Item No. 3. Mill the damp granules through a coarse screen (e.g., 1/4″) if needed. Dry the damp granules. Screen the dried granules if needed and mix with Item No. 4 and mix for 10-15 minutes. Add Item No. 5 and mix for 1-3 minutes. Compress the mixture to appropriate size and weight on a suitable tablet machine.

## Formulation VII: Capsules

| No. | Ingredient | mg/capsule | mg/capsule |
|---|---|---|---|
| 1. | Active compound | 100 | 500 |
| 2. | Lactose USP | 106 | 123 |
| 3. | Corn Starch, Food Grade | 40 | 70 |
| 4. | Magnesium Stearate NF | 4 | 7 |
| | Total | 250 | 700 |

Method of Manufacture

Mix Item Nos. 1, 2 and 3 in a suitable blender for 10-15 minutes. Add Item No. 4 and mix for 1-3 minutes. Fill the mixture into suitable two-piece hard gelatin capsules on a suitable encapsulating machine.

## Formulation VIII: Parenteral

| Ingredient | mg/vial | mg/vial |
|---|---|---|
| Active Compound Sterile Powder | 100 | 500 |

Add sterile water for injection or bacteriostatic water for injection, for reconstitution.

## Formulation VIX: Injectable

| Ingredient | mg/vial |
|---|---|
| Active Compound | 100 |
| Methyl p-hydroxybenzoate | 1.8 |
| Propyl p-hydroxybenzoate | 0.2 |
| Sodium Bisulfite | 3.2 |
| Disodium Edetate | 0.1 |
| Sodium Sulfate | 2.6 |
| Water for Injection q.s. ad | 1.0 ml |

Method of Manufacture

1. Dissolve parabens in a portion (85% of the final volume) of the water for injection at 65-70°C.
2. Cool to 25-35°C. Charge and dissolve the sodium bisulfite, disodium edetate and sodium sulfate.
3. Charge and dissolve drug.
4. Bring the solution to final volume by added water for injection.
5. Filter the solution through 0.22 membrane and fill into appropriate containers.
6. Terminally sterilize the units by autoclaving.

The quantity of active compound in a unit dose of preparation may be varied or adjusted from 1 mg to 100 mg according to the particular application and the potency of the active ingredient. The compositions can, if desired, also contain other therapeutic agents.

The amount of active compound applied to the involved lesions may be varied depending upon the requirements of the patient, the severity of the condition being treated and the particular compound being employed. Determination of the proper dosage for a particular situation is within the skill of the art. For convenience, the total daily dosage may be divided and administered in portions during the day if desired.

## Claims

1. Use of an azanaphthalene for preparing a medicament for treating hyperproliferative skin disease, said azanaphthalene having the structural formula Ia, Ib or Ic:

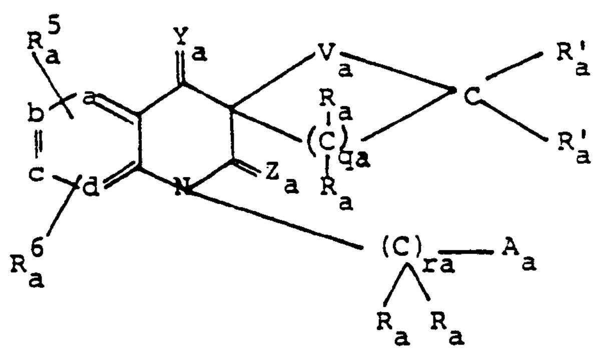

$I_a$

wherein:

two of the ring groups a, b, c and d may be CH or N and the remaining two groups represent CH;

$Y_a$ and $Z_a$ independently represent O or S;

$V_a$ represents O, $S(O)_{na}$, $N\text{-}R^8_a$ or

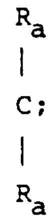

each $R_a$ independently represents hydrogen, alkyl having from 1 to 6 carbon atoms, $CH_2OH$, $COR^7_a$ (wherein $R^7_a$ represents hydrogen or alkyl having from 1 to 6 carbon atoms) or hydroxy, with the proviso that only one hydroxy group can be attached to one carbon atom;

each $R'_a$ independently is as defined for $R_a$ above, except that when $V_a$ represents O, $S(O)_{na}$ or $N\text{-}R^8_a$, $R'_a$ may not be hydroxy;

$R^8_a$ is hydrogen, alkyl having from 1 to 6 carbon atoms, carboxylic acyl having from 2 to 7 carbon atoms, alkylsulfonyl having from 1 to 6 carbon atoms, carboalkoxy having from 2 to 7 carbon atoms, $CONH_2$, phenyl or pyridyl of which the last two may be substituted with up to three substituents $Q_a$, whereby each $Q_a$ independently is hydroxy, alkyl having from 1 to 6 carbon atoms, halogen, nitro, alkoxy having from 1 to 6 carbon atoms, trifluoromethyl, cyano, cycloalkyl having from 3 to 7 carbon atoms, alkenyloxy having from 3 to 6 carbon atoms, alkynyloxy having from 3 to 6 carbon atoms, $S(O)_{na}\text{-}R^9_a$ {wherein na is as defined herein and $R^9_a$ is alkyl having from 1 to 6 carbon atoms}, $NHSO_2R^9_a$ {wherein $R^9_a$ is as defined above}, $NHSO_2CF_3$, $SO_2NH_2$, $COR^{10}_a$ {wherein $R^{10}_a$ is OH, $NH_2$ or $OR^9_a$ (wherein $R^9_a$ is as defined above)}, $O\text{-}B_a\text{-}COR^{10}_a$ {wherein $B_a$ is alkylene having from 1 to 4 carbon atoms and $R^{10}_a$ is as defined above}, or $NHCOR^{11}_a$ {wherein $R^{11}_a$ is hydrogen, alkyl having from 1 to 6 carbon atoms, alkoxy having from 1 to 6 carbon atoms, $COR^{12}_a$ (wherein $R^{12}_a$ is hydroxy or alkoxy having from 1 to 6 carbon atoms) or $NHR^{13}_a$ (wherein $R^{13}_a$ is hydrogen or alkyl having 1 to 6 carbon atoms)};

$R^5_a$ and $R^6_a$ may be the same or different and are hydrogen, alkyl having from 1 to 6 carbon atoms, halogen, nitro, alkoxy having from 1 to 6 carbon atoms, trifluoromethyl, alkylthio having 1 to 6 carbon atoms or cyano;

na is 0, 1 or 2;

ra is 0, 1 or 2;

qa is an integer of from 1 to 5; and

$A_a$ is phenyl, naphthyl, indenyl, indanyl, 2-, 3- or 4-pyridyl, 2-, 4-, 5- or 6-pyrimidyl, 2- or 3-pyrazinyl, 2- or 3-furyl, 2- or 3-thienyl, 2-, 4- or 5-imidazolyl, 2-, 4- or 5-thiazolyl or 2-, 4- or 5-oxazolyl, any of which may be substituted with up to three substituents $Q_a$ as defined herein above, or a solvate of such a compound of formula Ia;

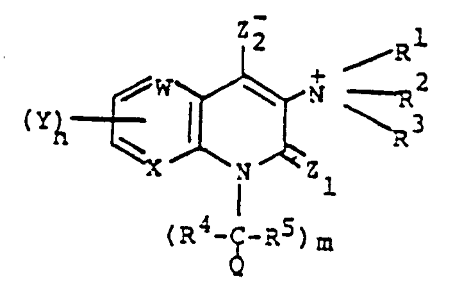

Ib

or pharmaceutical acceptable salts, solvates, or esters thereof, in a combination with a pharmaceutically acceptable carrier, wherein:

W and X may be the same or different and each independently represents -CH= or -N= ;

$Z_1$ and $Z_2$ are the same or different and each independently represents O or S ;

$R^1$, $R^2$, $R^3$, $R^4$ and $R^5$ are the same or different and each may be independently selected from the group consisting of H, alkyl having from 1 to 12 carbon atoms, alkenyl having from 3 to 8 carbon atoms, alkynyl having from 3 to 8 carbon atoms, alkoxyalkyl having from 1 to 6 carbon atoms in the alkoxy portion and from 2 to 6 atoms in the alkyl portion thereof, hydroxyalkyl having from 2 to 8 carbon atoms, cycloalkyl having from 3 to 8 carbon atoms, acyloxyalkyl having from 1 to 6 carbon atoms in the acyloxy portion and from 2 to 8 carbon atoms in the alkyl portion thereof, and $-R^6\text{-}CO_2R^0$ wherein $R^6$ represents an alkylene group having from 1 to 6 carbon atoms and $R^0$ represents hydrogen or an alkyl group having from 1 to 6 carbon atoms, with the provisos that in $R^1$, $R^2$, or $R^3$ the OH of the hydroxyalkyl group and the acyloxy of the acyloxyalkyl group are not joined to a carbon atom that is attached to the nitrogen atom to which $R^1$, $R^2$, and $R^3$ are attached and that, when $R^1$, $R^2$

and/or $R^3$ are alkenyl or alkynyl, there is at least one carbon-carbon single bond between the nitrogen atom to which $R^1$, $R^2$, and $R^3$ are attached and the carbon-carbon double or triple bond;

in addition, one of $R^1$, $R^2$ or $R^3$ can be phenyl which may be substituted with one to three substituents Y as defined below;

in further addition, two of $R^1$, $R^2$ and $R^3$ can be joined together to represent a ring which can contain from 2 to 8 carbon atoms, said ring optionally containing a -O-, -S- and/or $-NR^4-$ group in the ring (wherein $R^4$ is as defined above) and/or optionally containing a carbon-carbon double bond, and said ring optionally being substituted with one to three additional substituents $R^7$ which substituents may be the same or different and are each independently selected from OH with the proviso that OH is not on a carbon already joined to a hetero atom, acyloxy having from 1 to 6 carbon atoms, hydroxyalkyl having from 1 to 8 carbon atoms, alkoxyalkyl having from 1 to 6 carbon atoms in each of the alkoxy and alkyl portions thereof, alkyl having from 1 to 6 carbon atoms, alkenyl having from 3 to 8 carbon atoms, alkynyl having from 3 to 8 carbon atoms, or any two $R^7$ substituent groups may represent a hydrocarbon ring having from 4 to 8 total carbon atoms; and

in still further addition, all three of $R^1$, $R^2$ and $R^3$ together with the nitrogen atom to which they are attached can be joined together to represent a quinuclidine or aza-bicyclo[3.2.2]nonane ring, which ring can optionally be substituted by one to three substituent groups $R^7$ as defined above;

m is an integer of from 0 to 3;

n is an integer of from 0 to 2;

Q represents thiazolyl or phenyl optionally substituted with 1 to 3 substituents Y as defined below; and

each Y substituent is independently selected from the group consisting of hydroxy, alkyl having from 1 to 6 carbon atoms, halogen, $NO_2$, alkoxy having from 1 to 6 carbon atoms, trifluoromethyl, cyano, cycloalkyl having from 3 to 7 carbon atoms, alkenyloxy having from 3 to 6 carbon atoms, alkynyloxy having from 3 to 6 carbon atoms, hydroxyalkyl having from 1 to 6 carbon atoms, $-S(O)_n-R^8$ (wherein $R^8$ represents alkyl having from 1 to 6 carbon atoms and n is as defined above), $-SO_2NH_2$, $-CO-R^9$ (wherein $R^9$ represents OH, $-NH-R^8$ or $-O-R^8$, where $R^8$ is as defined above), $-O-B-COR^9$ (wherein B represents an alkylene group having from 1 to 4 carbon atoms and $R^9$ is as defined above), $-NH_2$, -NHCHO, $-NH-CO-R^9$ (wherein $R^9$ is as defined above, with the proviso that it is not hydroxy), $-NH-COCF_3$, $-NH-SO_2R^8$ (wherein $R^8$ is as defined above), and $-NHSO_2CF_3$ ;

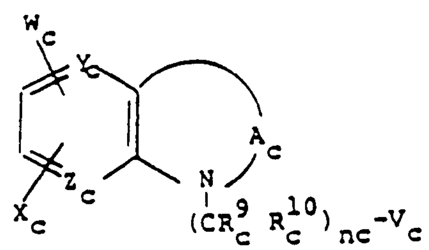

$I_c$

wherein:

$A_c$ is

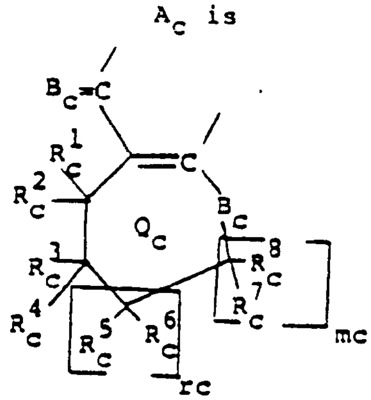

$A_c'$

or

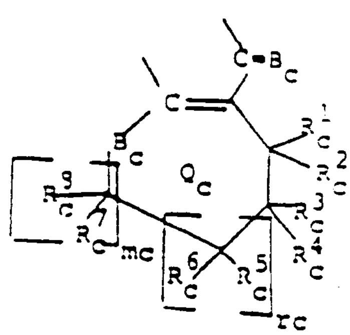

$A_c''$

$B_c$ is independently oxygen or sulfur;

$R^1_c$-$R^8_c$ may be the same or different and are hydrogen or alkyl having from 1 to 6 carbon atoms or two adjacent $R^1_c$-$R^8_c$ substituents may be combined to form an additional carbon to carbon bond;

rc and mc may be the same or different and are 0 or one;

the ring labeled $Q_c$ may optionally contain up to two additional double bonds;

nc is 0, 1 or 2;

$W_c$ and $X_c$ may be the same or different and are hydrogen, hydroxy, alkyl having from 1 to 6 carbon atoms, halogen, nitro, alkoxy having from 1 to 6 carbon atoms, tri-fluoromethyl, cyano, cycloalkyl having from 3 to 7 carbon atoms, alkenyloxy having from 3 to 6 carbon atoms, alkynyloxy having from 3 to 6 carbon atoms, $S(O)_{pc}$-$R^{11}{}_c$ {wherein pc is 0, 1 or 2 and $R^{11}{}_c$ is alkyl having from 1 to 6 carbon atoms}, $NHSO_2R^{11}{}_c$ {wherein $R^{11}{}_c$ is as defined above}, $NHSO_2CF_3$, $NHCOCF_3$, $SO_2NH_2$, $COR^{12}{}_c$ {wherein $R^{12}{}_c$ is OH, $NH_2$ or $OR^{11}{}_c$ (wherein $R^{11}{}_c$ is as defined above)}, O-$D_c$-$COR^{12}{}_c$ {wherein $D_c$ is alkylene having from 1 to 4 carbon atoms and $R^{12}{}_c$ is as defined above}, or $NHCOR^{13}{}_c$ {wherein $R^{13}{}_c$ is hydrogen, alkyl having from 1 to 6 carbon atoms, alkoxy having from 1 to 6 carbon atoms, $COR^{14}{}_c$ (wherein $R^{14}{}_c$ is hydroxy or alkoxy having from 1 to 6 carbon atoms) or $NHR^{15}{}_c$ (wherein $R^{15}{}_c$ is hydrogen or alkyl having from 1 to 6 carbon atoms)}, or phenoxy {wherein the benzene ring may be substituted with any of the other substituents $W_c$ and $X_c$};

$Y_c$ and $Z_c$ may be the same or different and are CH or N;

$V_c$ is phenyl, naphthyl, indenyl, indanyl, 2-, 3- or 4-pyridyl, 2-, 3- or 5-pyrimidinyl, 2- or 3-thienyl, 2- or 3-furyl or 2-, or 4- or 5-thiazolyl, any of which may be substituted with $W_c$ and $X_c$ as defined above; and $R^9{}_c$ and $R^{10}{}_c$ are independently hydrogen or alkyl having from 1 to 6 carbon atoms and solvates of such compounds.

2. The use according to claim 1 wherein in formula $I_a$, $Y_a$ and $Z_a$ are both oxygen and/or
ra is zero and/or
$R^5{}_a$ and $R^6{}_a$ are both hydrogen and/or
qa is 2, 3 or 4 and/or
$R_a$ and $R'_a$ independently are hydrogen, methyl, ethyl, propyl, isopropyl, n-butyl or iso-butyl and/or
$A_a$ is phenyl or phenyl substituted with one or two substituents $Q_a$ and/or
$V_a$ is O, $S(O)_{na}$ or N-$R^8{}_a$ preferably O.

3. The use according to claim 1 or 2 wherein in formula $I_a$ all of a, b, c, and d are CH or
a and d are both N and b and c are both CH or
d is N and a, b and c are CH.

4. The use according to claim 1 wherein the compound of formula $I_a$ is
1′-phenyl-spiro[cyclopentane-1,3′-(1-8)naphthyridine]-2′,4′-(1′H)-dione;
1′-phenyl-spiro[cyclopentane-1,3′-quinoline]-2′,4′-(1′H)-dione;
1′-(4-methylphenyl)spiro[cyclopentane-1,3′-(1,8)naphthyridine]-2′,4′-(1′H)-dione;
1′-(4-chlorophenyl)spiro[cyclopentane-1,3′-(1,8)naphthyridine]-2′,4′-(1′H)-dione;
1′-(3,4-dichlorophenyl)spiro[cyclopentane-1,3′-(1,8)naphthyridine]-2′,4′-(1′H)-dione;
1′-(3-chlorophenyl)spiro[cvclopentane-1,3′-(1,8)naphthyridine]-2′,4′-(1′H)-dione;
1′-(3-methoxyphenyl)spiro[cyclopentane-1,3′-(1,8)naphthyridine]-2′,4′-(1′H)-dione;
1′-(3-hydroxyphenyl)spiro[cyclopentane-1,3′-(1,8)naphthyridine]-2′,4′-(1′H)-dione;
1′-phenylspiro[cyclopentane-1,3′-quinoline]-2′,4′-(1′H)-dione;
1-phenyl-3′,4,′,5′,6′-tetrahydrospiro[1,8-naphthyridine-3,2′-(2H)pyran]-2,4-dione;
1-(3-methoxyphenyl)-3′,4′,5′,6′-tetrahydrospiro[1,8-naphthyridine-3,2′-(2H)pyran]-2,4-dione;
4,5-dihydro-1′-phenyl-spiro[furan-2(3H),3′(2′H) (1,8)naphthyridine]-2′,4′(1′H)-dione;
1-phenyl-spiro[1,8-naphthyridine-3,2′-oxetane]-2,4-dione; or
1-(3-chlorphenyl)-3′,4′,5′,6′-tetrahydrospiro[1,8-naphthyridine-3,2′-(2H)pyran]-2,4-dione or
solvate thereof.

5. The use according to claim 1 wherein the compound is of the formula

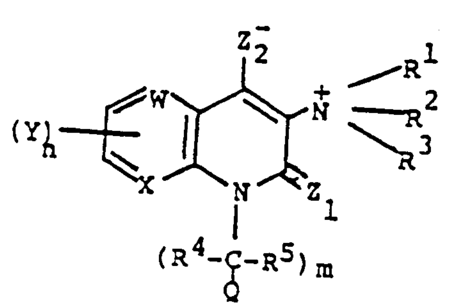

and pharmaceutically acceptable salts, esters, and solvates thereof, wherein:

W and X may be the same or different and each independently represents CH or N ;

$Z_1$ and $Z_2$ are the same or different and each independently represents O or S ;

$R^1$ , $R^2$, $R^3$, $R^4$ and $R^5$ are the same or different and each may be independently selected from the group consisting of H, alkyl having from 1 to 12 carbon atoms, alkenyl having from 3 to 8 carbon atoms, alkynyl having from 3 to 8 carbon atoms, alkoxyalkyl having from 1 to 6 carbon atoms in the alkoxy portion and from 2 to 6 atoms in the alkyl portion thereof, hydroxyalkyl having from 2 to 8 carbon atoms, cycloalkyl having from 3 to 8 carbon atoms, acyloxyalkyl having from 1 to 6 carbon atoms in the acyloxy portion and from 2 to 8 carbon atoms in the alkyl portion thereof, and $-R^6-CO_2R^0$ wherein $R^6$ represents an alkylene group having from 1 to 6 carbon atoms and $R^0$ represents hygrogen or an alkyl group having from 1 to 6 carbon atoms, with the provisos that in $R^1$, $R^2$, and $R^3$ the OH of the hydroxyalkyl group and the acyloxy of the acyloxyalkyl group are not joined to a carbon atom that is attached to the nitrogen atom to which $R^1$, $R^2$, and $R^3$ are attached and that, when $R^1$ , $R^2$ and/or $R^3$ are alkenyl or alkynyl, there is at least one carbon-carbon single bond between the nitrogen atom to which $R^1$, $R^2$, and $R^3$ are attached and the carbon-carbon double or triple bond;

in addition, one of $R^1$, $R^2$, or $R^3$ can be phenyl which may be substituted with one to three substituents Y as defined below;

in further addition, two of $R^1$, $R^2$ and $R^3$ can be joined together to represent a ring which can contain from 2 to 8 carbon atoms, said ring optionally containing a -O-, -S- and/or $-NR^4-$ group in the ring (wherein $R^4$ is as defined above) and/or optionally containing a carbon-carbon double bond, and said ring optionally being substituted with one to three additional substituents $R^7$ which substituents may be the same or different and are each independently selected from OH with the proviso that OH is not on a carbon already joined to a hetero atom, acyloxy having from 1 to 6 carbon atoms, hydroxyalkyl having from 1 to 8 carbon atoms, alkoxyalkyl having from 1 to 6 carbon atoms in each of the alkoxy and alkyl portions thereof, alkyl having from 1 to 6 carbon atoms, alkenyl having from 3 to 8 carbon toms, alkynyl having from 3 to 8 carbon atoms, or any two $R^7$ substituent groups may represent a hydrocarbon ring having from 4 to 8 total carbon atoms;

in still further addition, all three of $R^1$, $R^2$ and $R^3$ together with the nitrogen atom to which they are attached can be joined together to represent a quinuclidine or aza-bicyclo [3.2.2] nonane ring, which ring can optionally be substituted by one to three substituents groups $R^7$ as defined above;

m is an integer of from 0 to 3;

n is an integer of from 0 to 2;

Q represents thiazolyl or phenyl optionally substituted with 1 to 3 substituents Y as defined below;

each Y substituent is independently selected from the group consisting of hydroxy, alkyl having from 1 to 6 carbon atoms, halogen, $NO_2$, alkoxy having from 1 to 6 carbon atoms, trifluoromethyl, cyano, cycloalkyl having from 3 to 7 carbon atoms, alkenyloxy having from 3 to 6 carbon atoms, alkynyloxy having from 3 to 6 carbon atoms, hydroxyalkyl having from 1 to 6 carbon atoms, $-S(O)_n-R^8$ (wherein $R^8$ represents alkyl having from 1 to 6 carbon atoms and n is as defined above), $-SO_2NH_2$, $-CO-R^9$ (wherein $R^9$ represents OH, $-NH-R^8$ or $-O-R^8$, where $R^8$ is as defined above), $-O-B-COR^9$ (wherein B represents an alkylene group having from 1 to 4 carbon atoms and $R^9$ is as defined above), $-NH_2$, -NHCHO, $-NH-CO-R^9$ (wherein $R^9$ is as defined above, with the proviso that it is not hydroxy), $-NH-COCF_3$, $-NH-SO_2R^8$ (wherein $R^8$ is as defined above), and $-NHSO_2CF_3$; and

3-dimethylamino-4-hydroxy-1-phenyl-2-quinolone and 1-benzyl-3-dimethylamino-4-hydroxy-1-phenyl-2-quinolone are excluded.

6. The use according to claim 5, wherein in the formula at least one of W and X is -N=.

7. The use according to claim 6, wherein in the formula X is -N=, W is -CH= and $Z_1$ and $Z_2$ are both 0.

8. The use according to any one of claims 5 to 7 wherein in the formula n and m are 0.

9. The use according to claim 5 whereby the compound is selected from:

1(1,2-dihydro-4-hydroxy-1-phenyl-2-oxo-1,8-naphthyridin-3-yl)-1-methyl-pyrrolidinium hydroxide, inner salt;

1-(1,2-dihydro-4-hydroxy-1-phenyl-2-oxo-1,8-naphthyridin-3-yl)-pyrrolidinium hydroxide, inner salt;

1-(1,2-dihydro-4-hydroxy-1-phenyl-2-oxo-1,8-naphthyridin-3-yl)-4-hydroxy-piperidinium hydroxide, inner salt;

1-(1,2-dihydro-4-hydroxy-1-phenyl-2-oxo-1,8-naphthyridin-3-yl)-quinuclidinium hydroxide, inner salt;

1-(1,2-dihydro-4-hydroxy-1-phenyl-2-oxo-1,8-naphthyridin-3-yl)-1-methyl-morpholinium hydroxide, inner salt;

1-(1,2-dihydro-4-hydroxy-1-phenyl-2-oxo-1,8-naphthyridin-3-yl)-piperidinium hydroxide, inner salt; or

1-(1,2-dihydro-4-hydroxy-1-phenyl-2-oxo-1,8-naphthyridin-3-yl)-2-hydroxymethyl-piperidinium hydroxide, inner salt;

or pharmaceutically acceptable salts, esters, and solvates thereof.

10. The use according to claim 9 wherein the compound is 1-(1,2-dihydro-4-hydroxy-1-phenyl-2-oxo-1,8-naphthyridin-3-yl)-1-methyl-pyrrolidinium hydroxide, inner salt, and pharmaceutically acceptable salts, esters,

or solvates thereof.

11. The use according to claim 1 wherein in formula $I_c$ nc is zero and/or $Y_c$ is CH.

12. The use according to claim 1 wherein the compound of formula $I_c$ has the structural formula:

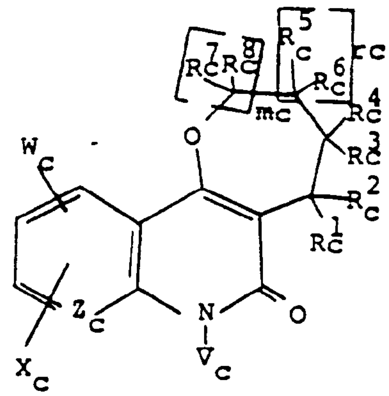

wherein $W_c$, $X_c$, $Z_c$, $V_c$, $R^1{}_c$ to $R^8{}_c$, rc and mc are as defined in claim 1
wherein preferably
$Z_c$ is N and/or
rc and mc are both zero or one or
the sum of rc and mc is one and/or
$B_c$ is oxygen and/or
$V_c$ is

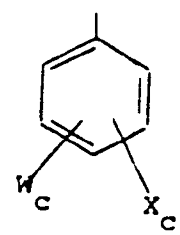

13. The use according to claim 1 wherein the compound of formula $I_c$ has the structural formula:

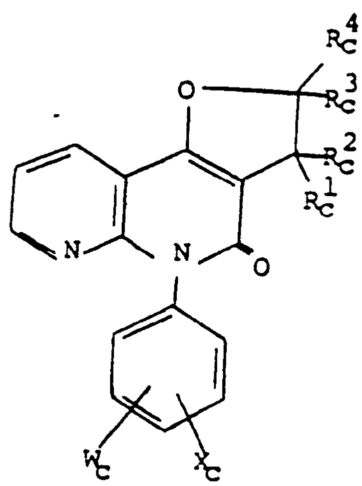

wherein $W_c$, $X_c$, $R^1{}_c$, $R^2{}_c$, $R^3{}_c$, and $R^4{}_c$ are as defined in claim 1, preferably $R^1{}_c$, $R^2{}_c$, $R^3{}_c$ and $R^4{}_c$ are hydrogen or methyl.

14. The use according to claim 1 wherein said compound of formula Ic has the structural formula:

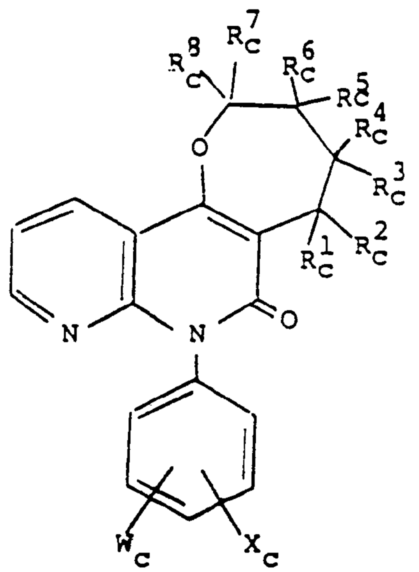

wherein $W_c$, $X_c$ and $R^1{}_c$-$R^8{}_c$ are as defined in claim 1, preferably
$R^1{}_c$-$R^8{}_C$ are hydrogen or methyl, especially hydrogen,
or one of $R^1{}_c$-$R^8{}_c$ is methyl and the rest are hydrogen and/or
$W_c$ is 3-chloro and $X_c$ is hydrogen, chlorine or fluorine or
$W_c$ is 3-methoxy and $X_c$ is hydrogen or fluorine or
$W_c$ and $X_c$ are both hydrogen.

15. The use according to claim 1 wherein said compound of formula $I_c$ has the structural formula:

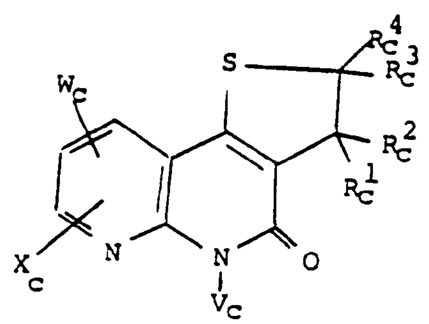

wherein $W_c$, $X_c$, $V_c$, $R^1{}_c$, $R^2{}_c$, $R^3{}_c$ and $R^4{}_c$ are as defined in claim 1.

16. The use according to claim 1 wherein said compound has the structural formula:

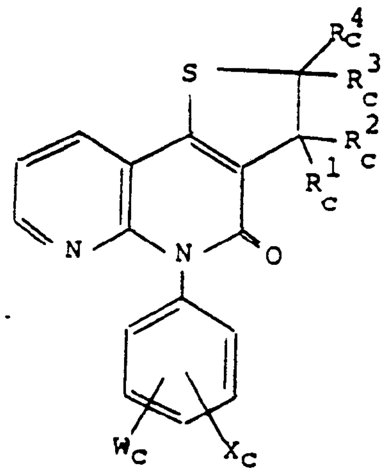

wherein $W_c$, $X_c$, $R^1_c$, $R^2_c$, $R^3_c$ and $R^4_c$ are as defined in claim 1, preferably
$W_c$ is 3-chloro and $X_c$ is hydrogen, chlorine or fluorine or
$W_c$ is 3-methoxy and $X_c$ is hydrogen or fluorine or
$W_c$ and $X_c$ are both hydrogen.

17. The use according to claim 1 wherein the compound of formula $I_c$ is
3,5-dihydro-5-phenyl-furo[3,2-c][1,8]naphthyridin-4[2H]-one;
3,5-dihydro-5-phenyl-thieno[3,2-c][1,8]-naphthyridin-4[2H]-one;
6-phenyl-2,3,4,6-tetrahydro-pyrano[3,2-c][1,8]-naphthyridin-5-one;
2-methyl-3,5-dihydro-5-phenyl-furo[3,2-c][1,8]-naphthyridin-4[2H]-one;
3,9-dihydro-9-phenyl-furo[2,3-b][1,8]naphthyridin-4[2H]-one;
3,9-dihydro-9-(p-methylphenyl)-furo[2,3-b][1,8]-naphthyridin-4[2H]-one;
3,9-dihydro-2-methyl-9-phenyl-furo[2,3-b][1,8]-naphthyridin-4[2H]-one;
3,5-dihydro-5-(p-methylphenyl)-furo[3,2-c][1,8]-naphthyridin-4[2H]-one;
3,5-dihydro-5-(p-fluorophenyl)-furo[3,2-c][1,8]-naphthyridin-4[2H]-one;
3,5-dihydro-5-(m-methoxyphenyl)-furo[3,2-c][1,8]-naphthyridin-4[2H]-one;
3,5-dihydro-5-(m-methylthiophenyl)-furo[3,2-c][1,8]-naphthyridin-4[2H]-one;
3,9-dihydro-9-(p-fluorophenyl)-furo[2,3-b][1,8]-naphthyridin-4[2H]-one;
3,9-dihydro-9-(m-methoxyphenyl)-furo[2,3-b][1,8]naphthyridin-4[2H]-one;
3,9-dihydro-9-(m-methylthiophenyl)-furo[3,2-c][1,8]-naphthyridin-4[2H]-one;3,5-dihydro-5-(3,4-dichlorophenyl)-furo[3,2-c][1,8]-naphthyridin-4[2H]-one;
3,5-dihydro-5-(3,4-dichlorophenyl)-2-methyl-furo [3,2-c][1,8]-naphthyridin-4[2H]-one;
3,5-dihydro-5-(4-chlorophenyl)-furo[3,2-c][1,8]-naphthyridin-4[2H]-one;
3,5-dihydro-5-(3-chlorophenyl)-furo[3,2-c][1,8]-naphthyridin-4[2H]-one;
3,5-dihydro-5-(3-chlorophenyl)-2-methyl-furo[3,2-c]-[1,8]-naphthyridin-4[2H]-one;
3,5-dihydro-5-(4-fluorophenyl)-2-methyl-furo[3,2-c]-[1,8]-naphthyridin-4[2H]-one;
3,5-dihydro-5-(3-methoxyphenyl)-2-methyl-furo[3,2-c][1,8]-naphthyridin-4[2H]-one;
3,5-dihydro-5-(3,5-dichlorophenyl)-furo[3,2-c][1,8]-naphthyridin-4[2H]-one;
3,5-dihydro-5-(3,5-dichlorophenyl)-2-methyl-furo[3,2-c][1,8]-naphthyridin-4[2H]-one;
3,5-dihydro-5-phenyl-2,2-dimethyl-furo[3,2-c][1,8]-naphthyridin-4[2H]-one;
3,5-dihydro-5-(3-methysulfonylaminophenyl)-2-methyl-furo[3,2-c][1,8]-naphthyridin-4[2H]-one;
3,5-dihydro-5-(3-methylsulfonylaminophenyl)-furo[3,2-c][1,8]-naphthyridin-4[2H]-one;
3,9-dihydro-9-(3,4-dichlorophenyl)-furo[2,3-b][1,8]-naphthyridin-4[2H]-one;
3,9-dihydro-9-(4-chlorophenyl)-furo[2,3-b][1,8]-naphthyridin-4[2H]-one;
3,9-dihydro-9-(3-chlorophenyl)-furo[2,3-b][1,8]-naphthyridin-4[2H]-one;
3,9-dihydro-9-(3-chlorophenyl)-2-methyl-furo[2,3-b][1,8]-naphthyridin-4[2H]-one;
3,9-dihydro-9-(4-fluorophenyl)-2-methyl-furo[2,3-b][1,8]-naphthyridin-4[2H]-one;
3,9-dihydro-9-(3-methoxyphenyl)-2-methyl-furo[2,3-b][1,8]-naphthyridin-4[2H]-one;
3,9-dihydro-9-(3,5-dichlorophenyl)-furo[2,3-b][1,8]-naphthyridin-4[2H]-one;
3,9-dihydro-9-(3,5-dichlorophenyl)-2-methyl-furo[2,3-b][1,8]-naphthyridin-4[2H]-one;
3,9-dihydro-9-(3-methylsulfonylaminophenyl)-2-methyl-furo[2,3-b][1,8]-naphthyridin-4[2H]-one;
6-(4-chlorophenyl)-2,3,4,6-tetrahydro-5H-pyrano[3,2-c]-[1,8]naphthyridin-5-one;

6-(3,4-dichlorophenyl)-2,3,4,6-tetrahydro-5H-pyrano [3,2-c][1,8]naphthyridin-5-one;
6-(4-methoxyphenyl)-2,3,4,6-tetrahydro-5H-pyrano [3,2-c][1,8]naphthyridin-5-one;
6-(4-methylphenyl)-2,3,4,6-tetrahydro-5H-pyrano [3,2-c][1,8]naphthyridin-5-one;
10-(3,4-dichlorophenyl)-2,3,4,10-tetrahydro-5H-pyrano-[2,3-b][1,8]naphthyridin-5-one;
10-(4-methoxyphenyl)-2,3,4,10-tetrahydro-5H-pyrano-[2,3-b][1,8]naphthyridin-5-one;
10-(4-chlorophenyl)-2,3,4,10-tetrahydro-5H-pyrano-[2,3-b][1,8]naphthyridin-5-one;
10-(4-methylphenyl)-2,3,4,10-tetrahydro-5H-pyrano-[2,3-b][1,8]naphthyridin-5-one;
10-phenyl-2,3,4,10-tetrahydro-5H-pyrano-[2,3-b][1,8]-naphthyridin-5-one;
7-phenyl-3,4,5,7-tetrahydro-oxepino-[3,2-c][1,8]-naphthyridin-6[2H]-one;
7-(4-chlorophenyl)-3,4,5,7-tetrahydro-oxepino-[3,2-c][1,8]-naphthyridin-6[2H]-one;
7-(3-chlorophenyl)-3,4,5,7-tetrahydro-oxepino-[3,2-c][1,8]-naphthyridin-6[2H]-one;
7-(3-methoxyphenyl)-3,4,5,7-tetrahydro-oxepino-[3,2-c][1,8]-naphthyridin-6[2H]-one; or
7-(3-hydroxyphenyl)-3,4,5,7-tetrahydro-oxepino[3,2-c][1,8]-naphthyridin-6[2H]-one.

## Patentansprüche

1. Verwendung eines Azanaphthalins zur Herstellung eines Medikaments zur Behandlung hyperproliferativer Hautkrankheiten, wobei das Azanaphthalin die Strukturformel Ia, Ib oder Ic hat:

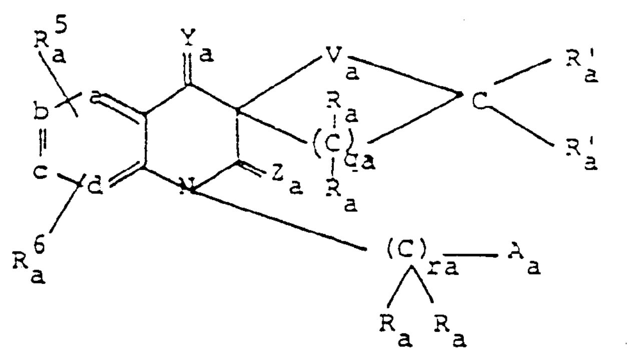

$I_a$

Worin:

zwei der Ringgruppen a, b, c oder d CH oder N sein können und die restlichen beiden Gruppen für CH stehen;

$Y_a$ und $Z_a$ unabhängig voneinander für O oder S stehen;

$V_a$ für O, $S(O)_{na}$, N-$R^8_a$ oder

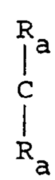

steht; $R_a$ jeweils unabhängig für Wasserstoff, Alkyl mit 1 bis 6 Kohlenstoff-Atomen, $CH_2OH$, $COR^7_a$ (worin $R^7_a$ Wasserstoff oder Alkyl mit 1 bis 6 Kohlenstoff-Atomen bedeutet) oder Hydroxy steht, mit der Maßgabe, daß nur eine Hydroxy-Gruppe an ein Kohlenstoff-Atom gebunden sein kann;

$R'_a$ jeweils unabhängig wie oben für $R_a$ definiert ist, außer, daß wenn $V_a$ O, $S(O)_{na}$, oder N-$R^8_a$ bedeutet, $R'_a$ nicht Hydroxy sein darf;

$R^8_a$ Wasserstoff, Alkyl mit 1 bis 6 Kohlenstoff-Atomen, Carbonsäureacyl mit 2 bis 7 Kohlenstoff-Atomen, Alkylsulfonyl mit 1 bis 6 Kohlenstoff-Atomen, Carbalkoxy mit 2 bis 7 Kohlenstoff-Atomen, $CONH_2$, Phenyl oder Pyridyl ist, wovon die beiden letzteren substituiert sein können mit bis zu drei Substituenten $Q_a$, wobei $Q_a$ unabhängig Hydroxy, Alkyl mit 1 bis 6 Kohlenstoff-Atomen, Halogen, Nitro, Alkoxy mit 1 bis 6 Kohlenstoff-Atomen, Trifluormethyl, Cyan, Cycloalkyl mit 3 bis 7 Kohlenstoff-Atomen, Alkenyloxy mit 3 bis 6 Kohlenstoff-Atomen, Alkinyloxy mit 3 bis 6 Kohlenstoff-Atomen, $S(O)_{na}$-$R^9_a$ (worin na wie hierin definiert ist, und $R^9_a$ Alkyl mit 1 bis 6 Kohlenstoff-Atomen ist), $NHSO_2R^9_a$ (worin $R^9_a$ wie oben definiert ist), $NHSO_2CF_3$, $SO_2NH_2$, $COR^{10}_a$ {worin

$R^{10}_a$ OH, $NH_2$ oder $OR^9_a$ ist (worin $R^9_a$ wie oben definiert ist)}, O-$B_a$-$COR^{10}_a$ (worin $B_a$ Alkylen mit 1 bis 4 Kohlenstoff-Atomen und $R^{10}_a$ wie oben definiert ist) oder $NHCOR^{11}_a$ ist {worin $R^{11}_a$ Wasserstoff, Alkyl mit 1 bis 6 Kohlenstoff-Atomen, Alkoxy mit 1 bis 6 Kohlenstoff-Atomen, $COR^{12}_a$ (worin $R^{12}_a$ Hydroxy oder Alkoxy mit 1 bis 6 Kohlenstoff-Atomen ist) oder $NHR^{13}_a$ (worin $R^{13}_a$ Wasserstoff oder Alkyl mit 1 bis 6 Kohlenstoff-Atomen ist)};

$R^5_a$ und $R^6_a$ gleich oder verschieden sein können und Wasserstoff, Alkyl mit 1 bis 6 Kohlenstoff-Atomen, Halogen, Nitro, Alkoxy mit 1 bis 6 Kohlenstoff-Atomen, Trifluormethyl, Alkylthio mit 1 bis 6 Kohlenstoff-Atomen oder Cyan sind;

na 0, 1 oder 2 ist;

ra 0, 1 oder 2 ist;

qa eine ganze Zahl von 1 bis 5 ist; und

$A_a$ Phenyl, Naphthyl, Indenyl, Indanyl, 2-, 3- oder 4-Pyridyl, 2-, 4-, 5- oder 6-Pyrimidyl, 2- oder 3-Pyrazinyl, 2- oder 3-Furyl, 2- oder 3-Thienyl, 2-, 4-oder 5-Imidazolyl, 2-, 4- oder 5-Thiazolyl oder 2-, 4-oder 5-Oxazolyl ist, die alle substituiert sein können mit bis zu drei Substituenten $Q_a$ wie hierin vorstehend definiert, oder ein Solvat einer solchen Verbindung der Formel Ia;

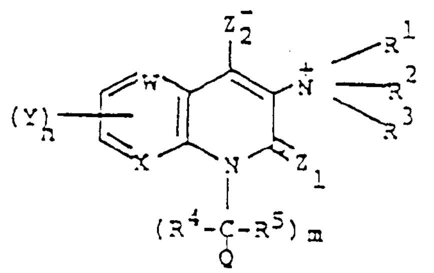

Ib

oder pharmazeutisch annehmbare Salze, Solvate oder Ester davon, in Kombination mit einem pharmazeutisch annehmbaren Träger, worin

W und X gleich oder verschieden sein können und jeweils unabhängig voneinander -CH= oder -N= bedeuten;

$Z_1$ und $Z_2$ gleich oder verschieden sind und jeweils unabhängig voneinander O oder S bedeuten;

$R^1$, $R^2$, $R^3$, $R^4$ und $R^5$ gleich oder verschieden sind und jeweils unabhängig voneinander ausgewählt sein können aus der Gruppe bestehend aus H, Alkyl mit 1 bis 12 Kohlenstoff-Atomen, Alkenyl mit 3 bis 8 Kohlenstoff-Atomen, Alkinyl mit 3 bis 8 Kohlenstoff-Atomen, Alkoxyalkyl mit 1 bis 6 Kohlenstoff-Atomen im Alkoxy-Teil und 2 bis 6 Kohlenstoff-Atomen im Alkyl-Teil, Hydroxyalkyl mit 2 bis 8 Kohlenstoff-Atomen, Cycloalkyl mit 3 bis 8 Kohlenstoff-Atomen, Acyloxyalkyl mit 1 bis 6 Kohlenstoff-Atomen im Acyloxy-Teil und 2 bis 8 Kohlenstoff-Atomen im Alkyl-Teil, und $-R^6-CO_2R^0$, worin $R^6$ eine Alkylen-Gruppe mit 1 bis 6 Kohlenstoff-Atomen bedeutet, und $R^0$ Wasserstoff oder eine Alkyl-Gruppe mit 1 bis 6 Kohlenstoff-Atomen bedeutet, mit der Maßgabe, daß in $R^1$, $R^2$ oder $R^3$ das OH der Hydroxyalkyl-Gruppe und das Acyloxy der Acyloxyalkyl-Gruppe nicht an ein Kohlenstoff-Atom gebunden sind, das an das Stickstoff-Atom gebunden ist, woran $R^1$, $R^2$ und $R^3$ geknüpft sind, und daß, wenn $R^1$, $R^2$ und/oder $R^3$ Alkenyl oder Alkinyl sind, wenigstens eine Kohlenstoff-Kohlenstoff-Einfachbindung besteht zwischen dem Stickstoff-Atom, woran $R^1$, $R^2$ und $R^3$ geknüpft sind, und der Kohlenstoff-Kohlenstoff-Doppel- oder -Dreifachbindung;

außerdem eines der $R^1$, $R^2$ und $R^3$ Phenyl sein kann, das mit einem bis drei Substituenten Y wie nachstehend definiert substituiert sein kann;

des weiteren zwei der $R^1$, $R^2$ und $R^3$ aneinander gebunden sein können, um einen Ring darzustellen, der 2 bis 8 Kohlenstoff-Atome enthalten kann, wobei der Ring gegebenenfalls eine -O-, -S- und/oder $-NR^4$-Gruppe im Ring enthält (worin $R^4$ wie oben definiert ist) und/oder gegebenenfalls eine Kohlenstoff-Kohlenstoff-Doppelbindung enthält, und der Ring gegebenenfalls substituiert ist mit einem bis drei zusätzlichen Substituenten $R^7$, die gleich oder verschieden sein können und jeweils unabhängig ausgewählt sind aus OH, mit der Maßgabe, daß sich OH nicht an einem bereits mit einem Heteroatom verknüpften Kohlenstoff befindet, Acyloxy mit 1 bis 6 Kohlenstoff-Atomen, Hydroxyalkyl mit 1 bis 8 Kohlenstoff-Atomen, Alkoxyalkyl mit 1 bis 6 Kohlenstoff-Atomen jeweils in dessen Alkoxy- oder Alkyl-Teil, Alkyl mit 1 bis 6 Kohlenstoff-Atomen, Alkenyl mit 3 bis 8 Kohlenstoff-Atomen, Alkinyl mit 3 bis 8 Kohlenstoff-Atomen, oder zwei beliebige $R^7$-Substituenten für einen Kohlenwasserstoff-Ring mit insgesamt 4 bis 8 Kohlenstoff-Atomen stehen können; und

des weiteren außerdem alle drei $R^1$, $R^2$ und $R^3$ zusammen mit dem Stickstoff-Atom, an welches sie gebunden sind, miteinander verknüpft sein können, um einen Chinuclidin- oder Azabicyclo[3.2.2]nonan-Ring darzustellen, der gegebenenfalls substituiert sein kann mit einer bis drei Substituenten-Gruppen $R^7$ wie oben definiert;

m eine ganze Zahl von 0 bis 3 ist;

n eine ganze Zahl von 0 bis 2 ist;

Q für Thiazolyl oder Phenyl steht, gegebenenfalls substituiert mit 1 bis 3 Substituenten Y wie nachstehend definiert; und

jeder Substituent Y unabhängig ausgewählt ist aus der Gruppe bestehend aus Hydroxy, Alkyl mit 1 bis 6 Kohlenstoff-Atomen, Halogen, $NO_2$, Alkoxy mit 1 bis 6 Kohlenstoff-Atomen, Trifluormethyl, Cyan, Cycloalkyl mit 3 bis 7 Kohlenstoff-Atomen, Alkenyloxy mit 3 bis 6 Kohlenstoff-Atomen, Alkinyloxy mit 3 bis 6 Kohlenstoff-Atomen, Hydroxyalkyl mit 1 bis 6 Kohlenstoff-Atomen, $-S(O)_n-R^8$ (worin $R^8$ Alkyl mit 1 bis 6 Kohlenstoff-Atomen bedeutet und n wie oben definiert ist), $-SO_2NH_2$, $-CO-R^9$ (worin $R^9$ für OH, $-NH-R^8$ oder $-O-R^8$ steht, worin $R^8$ wie oben definiert ist), $-O-B-COR^9$ (worin B für eine Alkylen-Gruppe mit 1 bis 4 Kohlenstoff-Atomen steht und $R^9$ wie oben definiert ist), $-NH_2$, -NHCHO, $-NH-CO-R^9$ (worin $R^9$ wie oben definiert ist, mit der Maßgabe, daß es nicht Hydroxy ist), $-NH-COCF_3$, $-NH-SO_2R^8$ (worin $R^8$ wie oben definiert ist) und $-NHSO_2CF_3$;

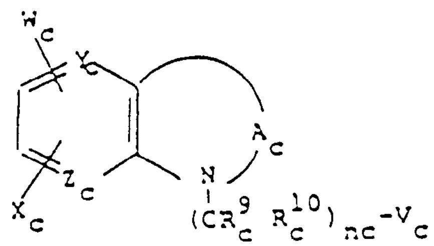

$I_c$

worin $A_c$

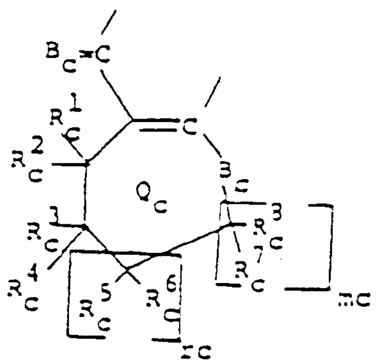

oder

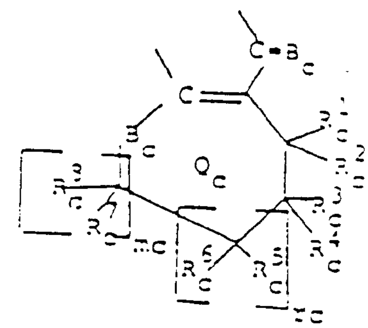

$A_c'$ $A_c''$

ist;

$B_c$ unabhängig Sauerstoff oder Schwefel ist;

$R^1_c$-$R^8_c$ gleich oder verschieden sein können und Wasserstoff oder Alkyl mit 1 bis 6 Kohlenstoff-Atomen sind, oder zwei benachbarte Substituenten $R^1_c$-$R^8_c$ kombiniert werden können, um eine zusätzliche Kohlenstoff-Kohlenstoff-Bindung zu bilden;

rc und mc gleich oder verschieden sein können und 0 oder 1 sind;

der mit $Q_c$ bezeichnete Ring gegebenenfalls bis zu zwei zusätzliche Doppelbindungen enthalten kann;

nc 0, 1 oder 2 ist;

$W_c$ und $X_c$ gleich oder verschieden sein können und Wasserstoff, Hydroxy, Alkyl mit 1 bis 6 Kohlenstoff-Atomen, Halogen, Nitro, Alkoxy mit 1 bis 6 Kohlenstoff-Atomen, Trifluormethyl, Cyan, Cycloalkyl mit 3 bis 7 Kohlenstoff-Atomen, Alkenyloxy mit 3 bis 6 Kohlenstoff-Atomen, Alkinyloxy mit 3 bis 6 Kohlenstoff-Atomen, $S(O)_{pc}-R^{11}_c$ (worin pc 0, 1 oder 2 ist, und $R^{11}_c$ Alkyl mit 1 bis 6 Kohlenstoff-Atomen ist), $NHSO_2R^{11}_c$ (worin $R^{11}_c$ wie oben definiert ist), $NHSO_2CF_3$, $NHCOCF_3$, $SO_2NH_2$, $COR^{12}_c$ {worin $R^{12}_c$ OH, $NH_2$ oder $OR^{11}_c$ ist (worin $R^{11}_c$ wie oben definiert ist)}, $O-D_c-COR^{12}_c$ (worin $D_c$ Alkylen mit 1 bis 4 Kohlenstoff-Atomen und $R^{12}_c$ wie oben definiert ist), oder $NHCOR^{13}_c$ ist {worin $R^{13}_c$ Wasserstoff, Alkyl mit 1 bis 6 Kohlenstoff-Atomen, Alkoxy mit 1 bis 6 Kohlenstoff-Atomen, $COR^{14}_c$ (worin $R^{14}_c$ Hydroxy oder Alkoxy mit 1 bis 6 Kohlenstoff-Atomen ist) oder $NHR^{15}_c$ (worin $R^{15}_c$ Wasserstoff oder Alkyl mit 1 bis 6 Kohlenstoff-Atomen ist)}, oder Phenoxy (worin der Benzol-Ring mit irgendeinem der anderen Substituenten $W_c$ und $X_c$ substituiert sein kann);

$Y_c$ und $Z_c$ gleich oder verschieden sein können und CH oder N sind;

$V_c$ Phenyl, Naphthyl, Indenyl, Indanyl, 2-, 3- oder 4-Pyridyl, 2-, 3- oder 5-Pyrimidinyl, 2- oder 3-Thienyl, 2-oder 3-Furyl oder 2-, 4- oder 5-Thiazolyl ist, die alle mit $W_c$ und $X_c$ wie oben definiert substituiert sein können; und $R^9{}_c$ und $R^{10}{}_c$ unabhängig voneinander Wasserstoff oder Alkyl mit 1 bis 6 Kohlenstoff-Atomen sind; und Solvate solcher Verbindungen.

2. Verwendung nach Anspruch 1, wobei $Y_a$ und $Z_a$ in Formel $I_a$ beide Sauerstoff sind und/oder
ra null ist und/oder
$R^5{}_a$ und $R^6{}_a$ beide Wasserstoff sind und/oder
qa 2, 3 oder 4 ist und/oder
$R_a$ und $R'_a$ unabhängig voneinander Wasserstoff, Methyl, Ethyl, Propyl, Isopropyl, n-Butyl oder Isobutyl sind und/oder
$A_a$ Phenyl oder mit einem oder zwei Substituenten $Q_a$ substituiertes Phenyl ist und/oder $V_a$ O, $S(O)_{na}$ oder N-$R^8{}_a$ ist, vorzugsweise O.

3. Verwendung nach Anspruch 1 oder 2, wobei in Formel $I_a$ alle a, b, c und d CH sind, oder
a und d beide N sind, und b und c beide CH sind, oder
d N ist, und a, b und c CH sind.

4. Verwendung nach Anspruch 1, wobei die Verbindung der Formel $I_a$ ist:
1′-Phenyl-spiro[cyclopentan-1,3′-(1,8)naphthyridin]-2′,4′(1′H)-dion;
1′-Phenyl-spiro[cyclopentan-1,3′-chinolin]-2′,4′(1′H)-dion;
1′-(4-Methylphenyl)spiro[cyclopentan-1,3′-(1,8)naphthyridin]-2′,4′(1′H)-dion;
1′-(4-Chlorphenyl)spiro[cyclopentan-1,3′-(1,8)naphthyridin]-2′,4′(1′H)-dion;
1′-(3,4-Dichlorphenyl)spiro[cyclopentan-1,3′-(1,8)naphthyridin]-2′,4′(1′H)-dion;
1′-(3-Chlorphenyl)spiro[cyclopentan-1,3′-(1,8)naphthyridin]-2′,4′(1′H)-dion;
1′-(3-Methoxyphenyl)spiro[cyclopentan-1,3′-(1,8)naphthyridin]-2′,4′(1′H)-dion;
1′-(3-Hydroxyphenyl)spiro[cyclopentan-1,3′-(1,8)naphthyridin]-2′,4′(1′H)-dion;
1-Phenyl-3′,4′,5′,6′-tetrahydrospiro[1,8-naphthyridin-3,2′-(2H)-pyran]-2,4-dion;
1-(3-Methoxyphenyl)-3′,4′,5′,6′-tetrahydrospiro[1,8-naphthyridin-3,2′-(2H)-pyran]-2,4-dion;
4,5-Dihydro-1′-phenyl-spiro[furan-2(3H),3′(2′H)-(1,8-naphthyridin)]-2′,4′(1′H)-dion;
1-Phenyl-spiro[1,8-naphthyridin-3,2′-oxetan]-2,4-dion; oder
1-(3-Chlorphenyl)-3′,4′,5′,6′-tetrahydrospiro[1,8-naphthyridin-3,2′-(2H)-pyran]-2,4-dion oder deren Solvate.

5. Verwendung nach Anspruch 1, wobei die Verbindung die Formel

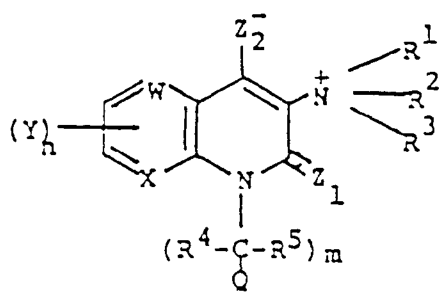

hat, und pharmazeutisch annehmbare Salze, Ester und Solvate davon, worin
W und X gleich oder verschieden sein können und jeweils unabhängig voneinander CH oder N bedeuten;
$Z_1$ und $Z_2$ gleich oder verschieden sind und jeweils unabhängig voneinander O oder S bedeuten;
$R^1$, $R^2$, $R^3$, $R^4$ und $R^5$ gleich oder verschieden sind und jeweils unabhängig voneinander ausgewählt sein können aus der Gruppe bestehend aus H, Alkyl mit 1 bis 12 Kohlenstoff-Atomen, Alkenyl mit 3 bis 8 Kohlenstoff-Atomen, Alkinyl mit 3 bis 8 Kohlenstoff-Atomen, Alkoxyalkyl mit 1 bis 6 Kohlenstoff-Atomen im Alkoxy-Teil und 2 bis 6 Kohlenstoff-Atomen im Alkyl-Teil, Hydroxyalkyl mit 2 bis 8 Kohlenstoff-Atomen, Cycloalkyl mit 3 bis 8 Kohlenstoff-Atomen, Acyloxyalkyl mit 1 bis 6 Kohlenstoff-Atomen im Acyloxy-Teil und 2 bis 8 Kohlenstoff-Atomen im Alkyl-Teil, und $-R^6-CO_2R^0$, worin $R^6$ eine Alkylen-Gruppe mit 1 bis 6 Kohlenstoff-Atomen bedeutet, und $R^0$ Wasserstoff oder eine Alkyl-Gruppe mit 1 bis 6 Kohlenstoff-Atomen bedeutet, mit der Maßgabe, daß in $R^1$, $R^2$ oder $R^3$ das OH der Hydroxyalkyl-Gruppe und das Acyloxy der Acyloxyalkyl-Gruppe nicht an ein Kohlenstoff-Atom gebunden sind, das an das Stickstoff-Atom gebunden ist, woran $R^1$, $R^2$ und $R^3$ geknüpft sind, und daß, wenn $R^1$, $R^2$ und/oder $R^3$ Alkenyl oder Alkinyl sind, wenigstens eine Kohlenstoff-Kohlenstoff-Einfachbindung besteht zwischen dem Stickstoff-Atom, woran $R^1$, $R^2$ und $R^3$ geknüpft sind, und der Kohlenstoff-Kohlenstoff-Doppel- oder -Dreifachbindung;

außerdem eines der $R^1$, $R^2$ und $R^3$ Phenyl sein kann, das mit einem bis drei Substituenten Y wie nachstehend definiert substituiert sein kann;

des weiteren zwei der $R^1$, $R^2$ und $R^3$ aneinander gebunden sein können, um einen Ring darzustellen, der 2 bis 8 Kohlenstoff-Atome enthalten kann, wobei der Ring gegebenenfalls eine -O-, -S- und/oder $-NR^4$-Gruppe im Ring enthält (worin $R^4$ wie oben definiert ist) und/oder gegebenenfalls eine Kohlenstoff-Kohlenstoff-Doppelbindung enthält, und der Ring gegebenenfalls substituiert ist mit einem bis drei zusätzlichen Substituenten $R^7$, die gleich oder verschieden sein können und jeweils unabhängig ausgewählt sind aus OH, mit der Maßgabe, daß sich OH nicht an einem bereits mit einem Heteroatom verknüpften Kohlenstoff befindet, Acyloxy mit 1 bis 6 Kohlenstoff-Atomen, Hydroxyalkyl mit 1 bis 8 Kohlenstoff-Atomen, Alkoxyalkyl mit 1 bis 6 Kohlenstoff-Atomen jeweils in dessen Alkoxy- oder Alkyl-Teil, Alkyl mit 1 bis 6 Kohlenstoff-Atomen, Alkenyl mit 3 bis 8 Kohlenstoff-Atomen, Alkinyl mit 3 bis 8 Kohlenstoff-Atomen, oder zwei beliebige $R^7$-Substituenten für einen Kohlenwasserstoff-Ring mit insgesamt 4 bis 8 Kohlenstoff-Atomen stehen können;

des weiteren außerdem alle drei $R^1$, $R^2$ und $R^3$ zusammen mit dem Stickstoff-Atom, an welches sie gebunden sind, miteinander verknüpft sein können, um einen Chinuclidin- oder Azabicyclo[3.2.2]nonan-Ring darzustellen, der gegebenenfalls substituiert sein kann mit einer bis drei Substituenten-Gruppen $R^7$ wie oben definiert;

m eine ganze Zahl von 0 bis 3 ist;

n eine ganze Zahl von 0 bis 2 ist;

Q für Thiazolyl oder Phenyl steht, gegebenenfalls substituiert mit 1 bis 3 Substituenten Y wie nachstehend definiert;

jeder Substituent Y unabhängig ausgewählt ist aus der Gruppe bestehend aus Hydroxy, Alkyl mit 1 bis 6 Kohlenstoff-Atomen, Halogen, $NO_2$, Alkoxy mit 1 bis 6 Kohlenstoff-Atomen, Trifluormethyl, Cyan, Cycloalkyl mit 3 bis 7 Kohlenstoff-Atomen, Alkenyloxy mit 3 bis 6 Kohlenstoff-Atomen, Alkinyloxy mit 3 bis 6 Kohlenstoff-Atomen, Hydroxyalkyl mit 1 bis 6 Kohlenstoff-Atomen, $-S(O)_n-R^8$ (worin $R^8$ Alkyl mit 1 bis 6 Kohlenstoff-Atomen bedeutet und n wie oben definiert ist), $-SO_2NH_2$, $-CO-R^9$ (worin $R^9$ für OH, $-NH-R^8$ oder $-O-R^8$ steht, worin $R^8$ wie oben definiert ist), $-O-B-COR^9$ (worin B für eine Alkylen-Gruppe mit 1 bis 4 Kohlenstoff-Atomen steht und $R^9$ wie oben definiert ist), $-NH_2$, -NHCHO, $-NH-CO-R^9$ (worin $R^9$ wie oben definiert ist, mit der Maßgabe, daß es nicht Hydroxy ist), $-NH-COCF_3$, $-NH-SO_2R^8$ (worin $R^8$ wie oben definiert ist) und $-NHSO_2CF_3$; und

3-Dimethylamino-4-hydroxy-1-phenyl-2-chinolon sowie 1-Benzyl-3-dimethylamino-4-hydroxy-1-phenyl-2-chinolon ausgeschlossen sind.

6. Verwendung nach Anspruch 5, wobei in der Formel wenigstens eines der W und X -N= ist.

7. Verwendung nach Anspruch 6, wobei in der Formel X -N= ist, W -CH= ist, und $Z_1$ und $Z_2$ beide O sind.

8. Verwendung nach irgendeinem der Ansprüche 5-7, wobei in der Formel n und m 0 sind.

9. Verwendung nach Anspruch 5, wobei die Verbindung ausgewählt ist aus:

1-(1,2-Dihydro-4-hydroxy-1-phenyl-2-oxo-1,8-naphthyridin-3-yl)-1-methyl-pyrrolidinium-hydroxid, inneres Salz;

1-(1,2-Dihydro-4-hydroxy-1-phenyl-2-oxo-1,8-naphthyridin-3-yl)-pyrrolidinium-hydroxid, inneres Salz;

1-(1,2-Dihydro-4-hydroxy-1-phenyl-2-oxo-1,8-naphthyridin-3-yl)-4-hydroxy-piperidinium-hydroxid, inneres Salz;

1-(1,2-Dihydro-4-hydroxy-1-phenyl-2-oxo-1,8-naphthyridin-3-yl)-chinuclidinium-hydroxid, inneres Salz;

1-(1,2-Dihydro-4-hydroxy-1-phenyl-2-oxo-1,8-naphthyridin-3-yl)-1-methyl-morpholinium-hydroxid, inneres Salz;

1-(1,2-Dihydro-4-hydroxy-1-phenyl-2-oxo-1,8-naphthyridin-3-yl)-piperidinium-hydroxid, inneres Salz; oder

1-(1,2-Dihydro-4-hydroxy-1-phenyl-2-oxo-1,8-naphthyridin-3-yl)-2-hydroxymethyl-piperidinium-hydroxid, inneres Salz;

oder deren pharmazeutisch annehmbaren Salzen, Estern und Solvaten.

10. Verwendung nach Anspruch 9, wobei die Verbindung 1-(1,2-Dihydro-4-hydroxy-1-phenyl-2-oxo-1,8-naphthyridin-3-yl)-1-methyl-pyrrolidinium-hydroxid, inneres Salz, ist und deren pharmazeutisch annehmbare Salze, Ester oder Solvate.

11. Verwendung nach Anspruch 1, wobei in der Formel $I_c$ nc null ist, und/oder $Y_c$ CH ist.

12. Verwendung nach Anspruch 1, wobei die Verbindung der Formel $I_c$ die Strukturformel

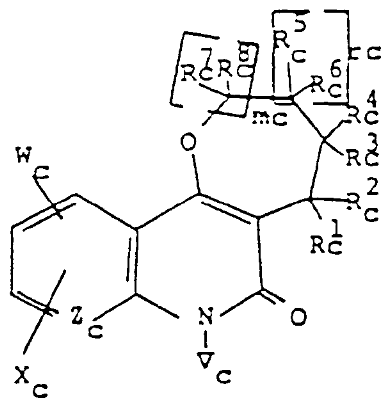

hat, worin $W_c$, $X_c$, $Z_c$, $V_c$, $R^1_c$ bis $R^8_c$, rc und mc wie in Anspruch 1 definiert sind, worin vorzugsweise
$Z_c$ N ist, und/oder
rc und mc beide null oder eins sind, oder die Summe von rc und mc eins ist, und/oder
$B_c$ Sauerstoff ist, und/oder
$V_c$

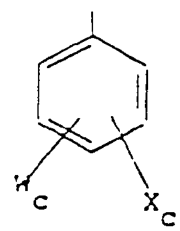

ist.

13. Verwendung nach Anspruch 1, wobei die Verbindung der Formel $I_c$ die Strukturformel

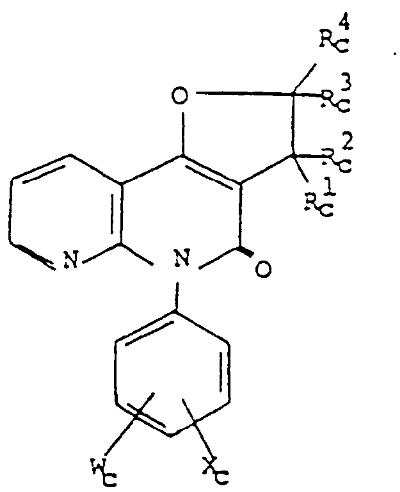

hat, worin $W_c$, $X_c$, $R^1_c$, $R^2_c$, $R^3_c$ und $R^4_c$ wie in Anspruch 1 definiert sind, und $R^1_c$, $R^2_c$, $R^3_c$ und $R^4_c$ vorzugsweise Wasserstoff oder Methyl sind.

14. Verwendung nach Anspruch 1, wobei die Verbindung der Formel $I_c$ die Strukturformel

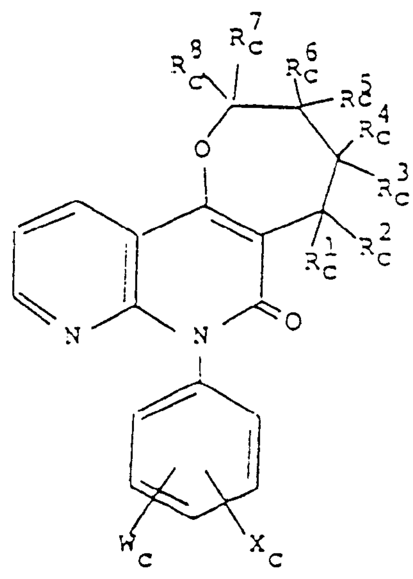

hat, worin $W_c$, $X_c$, und $R^1{}_c$-$R^8{}_c$ wie in Anspruch 1 definiert sind, und vorzugsweise $R^1{}_c$-$R^8{}_c$ Wasserstoff oder Methyl sind, insbesondere Wasserstoff;
oder eines der $R^1{}_c$-$R^8{}_c$ Methyl ist und der Rest Wasserstoff; und/oder
$W_c$ 3-Chlor ist, und $X_c$ Wasserstoff, Chlor oder Fluor ist; oder
$W_c$ 3-Methoxy ist, und $X_c$ Wasserstoff oder Fluor ist; oder
$W_c$ und $X_c$ beide Wasserstoff sind.

15. Verwendung nach Anspruch 1, wobei die Verbindung der Formel $I_c$ die Strukturformel

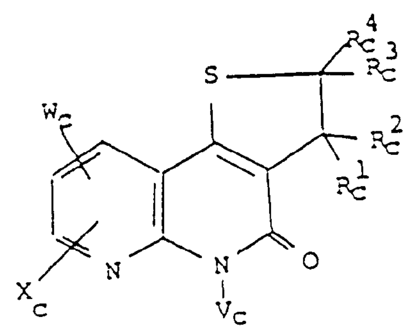

hat, worin $W_c$, $X_c$, $V_c$, $R^1{}_c$, $R^2{}_c$, $R^3{}_c$ und $R^4{}_c$ wie in Anspruch 1 definiert sind.

16. Verwendung nach Anspruch 1, wobei die Verbindung die Strukturformel

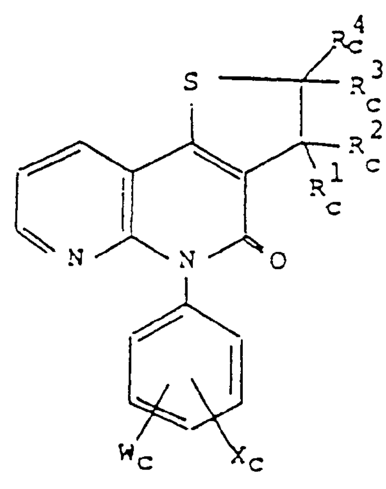

hat, worin $W_c$, $X_c$, $R^1{}_c$, $R^2{}_c$, $R^3{}_c$ und $R^4{}_c$ wie in Anspruch 1 definiert sind, und vorzugsweise
$W_c$ 3-Chlor ist, und $X_c$ Wasserstoff, Chlor oder Fluor ist; oder
$W_c$ 3-Methoxy ist, und $X_c$ Wasserstoff oder Fluor ist; oder
$W_c$ und $X_c$ beide Wasserstoff sind.

17. Verwendung nach Anspruch 1, wobei die Verbindung der Formel $I_c$ ist:
3,5-Dihydro-5-phenyl-furo[3,2-c][1,8]naphthyridin-4(2H)-on;
3,5-Dihydro-5-phenyl-thieno[3,2-c][1,8]naphthyridin-4(2H)-on;
6-Phenyl-2,3,4,6-tetrahydro-pyrano-[3,2-c][1,8]naphthyridin-5-on;
2-Methyl-3,5-dihydro-5-phenyl-furo[3,2-c][1,8]naphthyridin-4(2H)-on;
3,9-Dihydro-9-phenyl-furo[2,3-b][1,8]naphthyridin-4(2H)-on;
3,9-Dihydro-9-(p-methylphenyl)-furo[2,3-b][1,8]naphthyridin-4(2H)-on;
3,9-Dihydro-2-methyl-9-phenyl-furo[2,3-b][1,8]naphthyridin-4(2H)-on;
3,5-Dihydro-5-(p-methylphenyl)-furo[3,2-c][1,8]naphthyridin-4(2H)-on;
3,5-Dihydro-5-(p-fluorphenyl)-furo[3,2-c][1,8]naphthyridin-4(2H)-on;
3,5-Dihydro-5-(p-methoxyphenyl)-furo[3,2-c][1,8]naphthyridin-4(2H)-on;
3,5-Dihydro-5-(m-methylphenyl)-furo[3,2-c][1,8]naphthyridin-4(2H)-on;
3,9-Dihydro-9-(p-fluorphenyl)-furo[2,3-b][1,8]naphthyridin-4(2H)-on;
3,9-Dihydro-9-(m-methoxyphenyl)-furo[2,3-b][1,8]naphthyridin-4(2H)-on;
3,9-Dihydro-9-(m-methylthiophenyl)-furo[3,2-c][1,8]naphthyridin-4(2H)-on;
3,5-Dihydro-5-(3,4-dichlorphenyl)-furo[3,2-c][1,8]naphthyridin-4(2H)-on;
3,5-Dihydro-5-(3,4-dichlorphenyl)-2-methyl-furo[3,2-c][1,8]naphthyridin-4(2H)-on;
3,5-Dihydro-5-(4-chlorphenyl)-furo[3,2-c][1,8]naphthyridin-4(2H)-on;
3,5-Dihydro-5-(3-chlorphenyl)-furo[3,2-c][1,8]naphthyridin-4(2H)-on;
3,5-Dihydro-5-(3-chlorphenyl)-2-methyl-furo[3,2-c][1,8]naphthyridin-4(2H)-on;
3,5-Dihydro-5-(4-fluorphenyl)-2-methyl-furo[3,2-c][1,8]naphthyridin-4(2H)-on;
3,5-Dihydro-5-(3-methoxyphenyl)-2-methyl-furo[3,2-c][1,8]naphthyridin-4(2H)-on;
3,5-Dihydro-5-(3,5-dichlorphenyl)-furo[3,2-c][1,8]naphthyridin-4(2H)-on;
3,5-Dihydro-5-(3,5-dichlorphenyl)-2-methyl-furo[3,2-c][1,8]naphthyridin-4(2H)-on;
3,5-Dihydro-5-phenyl-2,2-dimethyl-furo[3,2-c][1,8]naphthyridin-4(2H)-on;
3,5-Dihydro-5-(3-methylsulfonylaminophenyl)-2-methyl-furo[3,2-c][1,8]naphthyridin-4(2H)-on;
3,5-Dihydro-5-(3-methylsulfonylaminophenyl)-furo[3,2-c][1,8]naphthyridin-4(2H)-on;
3,9-Dihydro-9-(3,4-dichlorphenyl)-furo[2,3-b][1,8]naphthyridin-4(2H)-on;
3,9-Dihydro-9-(4-chlorphenyl)-furo[2,3-b][1,8]naphthyridin-4(2H)-on;
3,9-Dihydro-9-(3-chlorphenyl)-furo[2,3-b][1,8]naphthyridin-4(2H)-on;
3,9-Dihydro-9-(3-chlorphenyl)-2-methyl-furo[2,3-b][1,8]naphthyridin-4(2H)-on;
3,9-Dihydro-9-(4-fluorphenyl)-2-methyl-furo[2,3-b][1,8]naphthyridin-4(2H)-on;
3,9-Dihydro-9-(3-methoxyphenyl)-2-methyl-furo[2,3-b][1,8]naphthyridin-4(2H)-on;
3,9-Dihydro-9-(3,5-dichlorphenyl)-furo[2,3-b][1,8]naphthyridin-4(2H)-on;
3,9-Dihydro-9-(3,5-dichlorphenyl)-2-methyl-furo[2,3-b][1,8]naphthyridin-4(2H)-on;
3,9-Dihydro-9-(3-methylsulfonylaminophenyl)-2-methyl-furo[2,3-b][1,8]naphthyridin-4(2H)-on;
6-(4-Chlorphenyl)-2,3,4,6-tetrahydro-5H-pyrano[3,2-c][1,8]naphthyridin-5-on;
6-(3,4-Dichlorphenyl)-2,3,4,6-tetrahydro-5H-pyrano[3,2-c][1,8]naphthyridin-5-on;
6-(4-Methoxyphenyl)-2,3,4,6-tetrahydro-5H-pyrano[3,2-c][1,8]naphthyridin-5-on;
6-(4-Methylphenyl)-2,3,4,6-tetrahydro-5H-pyrano[3,2-c][1,8]naphthyridin-5-on;
10-(3,4-Dichlorphenyl)-2,3,4,10-tetrahydro-5H-pyrano[2,3-b][1,8]naphthyridin-5-on;
10-(4-Methoxyphenyl)-2,3,4,10-tetrahydro-5H-pyrano[2,3-b][1,8]naphthyridin-5-on;
10-(4-Chlorphenyl)-2,3,4,10-tetrahydro-5H-pyrano[2,3-b][1,8]naphthyridin-5-on;
10-(4-Methylphenyl)-2,3,4,10-tetrahydro-5H-pyrano[2,3-b][1,8]naphthyridin-5-on;
10-Phenyl-2,3,4,10-tetrahydro-5H-pyrano[2,3-b][1,8]naphthyridin-5-on;
7-Phenyl-3,4,5,7-tetrahydro-oxepino[3,2-c][1,8]naphthyridin-6(2H)-on;
7-(4-Chlorphenyl)-3,4,5,7-tetrahydro-oxepino[3,2-c][1,8]naphthyridin-6(2H)-on;
7-(3-Chlorphenyl)-3,4,5,7-tetrahydro-oxepino[3,2-c][1,8]naphthyridin-6(2H)-on;
7-(3-Methoxyphenyl)-3,4,5,7-tetrahydro-oxepino[3,2-c][1,8]naphthyridin-6(2H)-on; oder
7-(3-Hydroxyphenyl)-3,4,5,7-tetrahydro-oxepino[3,2-c][1,8]naphthyridin-6(2H)-on.

## Revendications

1. Utilisation d'un azanaphtalène pour la préparation d'un médicament pour le traitement d'une maladie hyperproliférante de la peau, ledit azanaphtalène ayant la formule de structure Ia, Ib ou Ic:

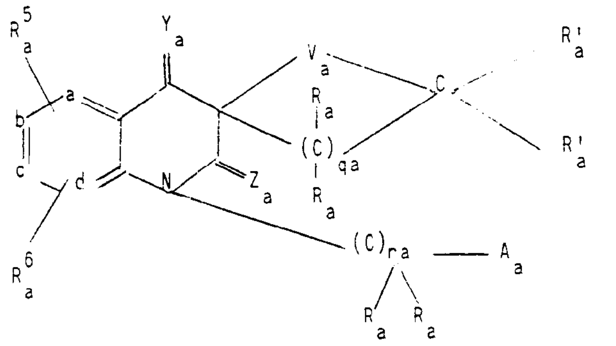

Ia

dans laquelle:

deux des groupes de noyau a,b,c ou d peuvent être CH ou N et les deux groupes restants représentent CH;

$Y_a$ et $Z_a$ représentent indépendamment O ou S;

$V_a$ représente O,$S(O)_{na}$, N-$R^8_a$ ou

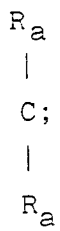

chaque $R_a$ représente indépendamment hydrogène, alkyle ayant 1 à 6 atomes de carbone, $CH_2OH$, $COR^7_a$ (où $R^7_a$ représente hydrogène ou alkyle ayant 1 à 6 atomes de carbone) ou hydroxy, à condition que seul un groupe hydroxy puisse être attaché à un atome de carbone;

chaque $R'_a$ est indépendamment tel que défini pour $R_a$ ci-dessus à l'exception que lorsque $V_a$ représente O,$S(O)_{na}$ ou N-$R^8_a$ , $R'_a$ peut ne pas être hydroxy;

$R^8_a$ est hydrogène, alkyle ayant 1 à 6 atomes de carbone, acyle carboxylique ayant 2 à 7 atomes de carbone, alkylsulfonyle ayant 1 à 6 atomes de carbone, carboalcoxy ayant 2 à 7 atomes de carbone, $CONH_2$, phényle ou pyridyle, ces deux derniers pouvant être substitués par jusqu'à trois substituants $Q_a$, chaque $Q_a$ étant indépendamment hydroxy, alkyle ayant 1 à 6 atomes de carbone, halogène, nitro, alcoxy ayant 1 à 6 atomes de carbone, trifluorométhyle, cyano, cycloalkyle ayant 3 à 7 atomes de carbone, alcényloxy ayant 3 à 6 atomes de carbone, alkynyloxy ayant 3 à 6 atomes de carbone, $S(O)_{na}$-$R^9$ {où na est tel que défini ici et $R^9_a$ est alkyle ayant 1 à 6 atomes de carbone}, $NHSO_2R^9_a$ {où $R^9$ est tel que défini ci-dessus}, $NHSO_2CF_3$, $SO_2NH_2$, $COR^{10}_a$ {où $R^{10}$ est OH, $NH_2$ ou $OR^9_a$ (où $R^9_a$ est tel que défini ci-dessus)}, O-$B_a$ -$COR^{10}_a$ { où $B_a$ est alkylène ayant 1 à 4 atomes de carbone et $R^{10}_a$ est tel que défini ci-dessus}, où $NHCOR^{11}$ { où $R^{11}_a$ est hydrogène, alkyle ayant 1 à 6 atomes de carbone, alcoxy ayant 1 à 6 atomes de carbone, $COR^{12}_a$ ( où $R^{12}_a$ est hydroxy ou alcoxy ayant 1 à 6 atomes de carbone) ou $NHR^{13}_a$ (où $R^{13}_a$ est hydrogène ou alkyle ayant 1 à 6 atomes de carbone)};

$R^5_a$ est $R^6_a$ peuvent être identiques ou différents et sont hydrogène, alkyle ayant 1 à 6 atomes de carbone, halogène, nitro, alcoxy ayant 1 à 6 atomes de carbone, trifluorométhyle, alkylthio ayant 1 à 6 atomes de carbone ou cyano;

na est O, 1 ou 2;

ra est O, 1 ou 2;

qa est un nombre entier de 1 à 5; et

$A_a$ est phényle, naphtyle, indényle, indanyle, 2-, 3- ou 4- pyridyle, 2-, 4-, 5- ou 6-pyrimidyle, 2- ou 3-pyrazinyle, 2- ou 3-furyle, 2- ou 3-thiényle, 2-, 4- ou 5- imidazolyle, 2-, 4- ou 5-thiazolyle ou 2-, 4- ou 5-oxazolyle, chacun d'entre eux pouvant être substitué par trois substituants $Q_a$ tels que définis précédemment, ou bien un

solvat d'un tel composé de formule Ia;

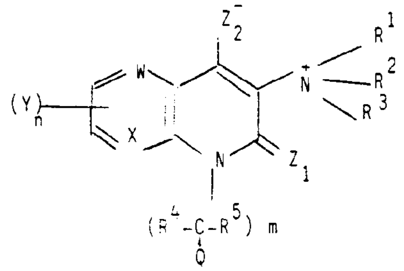

$I_b$

ou ses sels, solvats ou esters acceptables en pharmacie, en combinaison avec un support acceptable en pharmacie, dans laquelle:

W et X peuvent être identiques ou différents et chacun représente indépendamment -CH= ou -N=;

$Z_1$ et $Z_2$ sont identiques ou différents et chacun représente indépendamment O ou S;

$R^1$, $R^2$, $R^3$, $R^4$, et $R^5$ sont identiques ou différents et chacun peut être indépendamment choisi dans le groupe consistant en H, alkyle ayant 1 à 12 atomes de carbone, alkényle ayant 3 à 8 atomes de carbone, alkynyle ayant 3 à 8 atomes de carbone, alcoxyalkyle ayant 1 à 6 atomes de carbone à la portion alcoxy et 2 à 6 atomes dans sa portion alkyle, hydroxyalkyle ayant 2 à 8 atomes de carbone, cycloalkyle ayant 3 à 8 atomes de carbone, alcyloxyalkyle ayant 1 à 6 atomes de carbone dans sa portion acyloxy et 2 à 8 atomes de carbone dans la portion alkyle et $-R^6-CO_2R^0$ où $R^6$ représente un groupe alkylène ayant 1 à 6 atomes de carbone et $R^0$ représente hydrogène ou un groupe alkyle ayant 1 à 6 atomes de carbone, aux conditions que dans $R^1$, $R^2$ ou $R^3$, OH du groupe hydroxyalkyle et l'acyloxy du groupe acyloxyalkyle ne soient pas joints à un atome de carbone qui est attaché à l'atome d'azote auquel $R^1$, $R^2$ et $R^3$ sont attachés et que, quand $R^1$, $R^2$ et/ou $R^3$ sont alcényles ou alkynyle, il y ait au moins une simple liaison carbone-carbone entre l'atome d'azote auquel $R^1$, $R^2$ et $R^3$ sont attachés et la double ou triple liaison carbone-carbone;

de plus, l'un de $R^1$, $R^2$ ou $R^3$ peut être phényle qui peut être substitué par un à trois substituants Y tels que définis ci-dessous;

de plus encore, deux de $R^1$, $R^2$ et $R^3$ peuvent être joints pour représenter un noyau qui peut contenir 2 à 8 atomes de carbone, ledit noyau contenant facultativement un groupe -O-, -S- et/ou $-NR^4-$ dans le noyau (où R4 est tel que défini ci-dessus) et/ou contenant facultativement une double liaison carbone-carbone et ledit noyau étant facultativement substitué par un à trois substituants additionnels $R^7$, lesquels substituants peuvent être identiques ou différents et chacun est indépendamment choisi parmi OH à condition que OH ne soit pas sur un carbone déjà joint à un hétéroatome, acyloxy ayant 1 à 6 atomes de carbone, hydroxyalkyle ayant 1 à 8 atomes ce carbone, alcoxyalkyle ayant 1 à 6 atomes de carbone dans chacune de ses portions alcoxy et alkyle, alkyle ayant 1 à 6 atomes de carbone, alcényle ayant 3 à 8 atomes de carbone, alkynyle ayant 3 à 8 atomes de carbone, ou bien deux groupes substituants $R^7$ peuvent représenter un noyau d'hydrocarbure ayant 4 à 8 atomes de carbone au total; et

de plus encore, les trois $R^1$, $R^2$ et $R^3$ avec l'atome d'azote auquel ils sont attachés peuvent être joints pour représenter un noyau quinuclidine ou-aza bicyclo {3.2.2}nonane, lequel noyau peut facultativement être substitué par un à trois groupes substituants $R^7$ tels que définis ci-dessus;

m est un nombre entier de 0 à 3;

n est un nombre entier de 0 à 2;

Q représente thiazolyle ou phényle facultativement substitué par 1 à 3 substituants Y tels que définis ci-dessous; et

chaque substituant Y est indépendamment choisi dans le groupe consistant en hydroxy, alkyle ayant 1 à 6 atomes de carbone, halogène, $NO_2$, alcoxy ayant 1 à 6 atomes de carbone, trifluorométhyle, cyano, cycloalkyle ayant 3 à 7 atomes de carbone, alcényloxy ayant 3 à 6 atomes de carbone, alkynyloxy ayant 3 à 6 atomes de carbone, hydroxyalkyle ayant 1 à 6 atomes de carbone, $-S(O)_n-R^8$ (où $R^8$ représente alkyle ayant 1 à 6 atomes de carbone et n est tel que défini ci-dessus), $-SO_2NH^2$, $-CO-R^9$ (où $R^9$ représente OH, $-NH-R^8$ ou $-O-R^8$,où $R^8$ est tel que défini ci-dessus), $-O-B-COR^9$ (où B représente un groupe alkylène ayant 1 à 4 atomes de carbone et $R^9$ est tel que défini ci-dessus), $-NH_2$, -NHCHO, $-NH-CO-R^9$ (où $R^9$ est tel que défini ci-dessus, à condition qu'il ne soit pas hydroxy), $-NH-COCF_3$, $-NH-SO_2R^8$ (où $R^8$ est tel que défini ci-dissus), et $-NHSO_2CF_3$;

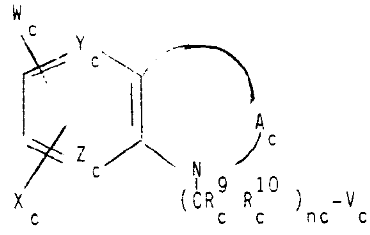

$I_c$

dans laquelle:

$A_c$ est

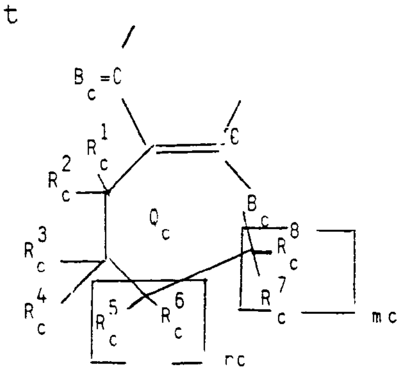

ou

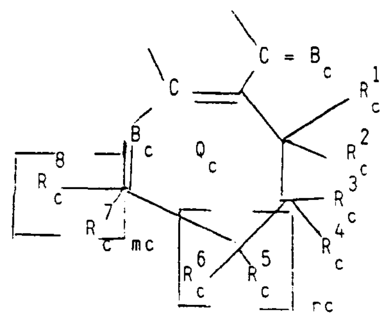

$A_c{}'$ $A_c{}''$

Br est indépendamment oxygène ou soufre;

$R^1_c$ -$R^8_c$ peuvent être identiques ou différents et sont hydrogène ou alkyle ayant 1 à 6 atomes de carbone ou bien deux substituants $R^1_c$ -$R^8_c$ adjacents peuvent être combinés pour former une liaison additionnelle carbone à carbone;

rc et mc peuvent être identiques ou différents et sont 0 ou 1;

le noyau marqué $Q_c$ peut facultativement contenir jusqu'à deux doubles liaisons additionnelles;

nc est 0, 1 ou 2;

$W_c$ et $X_c$ peuvent être identiques ou différents et sont hydrogène, hydroxy, alkyle ayant 1 à 6 atomes de carbone, halogène, nitro, alcoxy ayant 1 à 6 atomes de carbone, trifluorométhyle, cyano, cycloalkyle ayant 3 à 7 atomes de carbone, alcényloxy ayant 3 à 6 atomes de carbone, alkynyloxy ayant 3 à 6 atomes de carbone, $S(O)_{pc}$-$R^{11}_c$ {où pc est 0, 1 ou 2 et $R^{11}_c$ est alkyle ayant 1 à 6 atomes de carbone}, $NHSO_2R^{11}_c$ { où $R^{11}_c$ est tel que défini ci-dessus}, $NHSO_2CF_3$, $NHCOCF_3$, $SO_2NH_2$, $COR^{12}_c$ {où $R^{12}_c$ est OH, $NH_2$ ou $OR^{11}_c$ (où $R^{11}$ est tel que défini ci-dessus }, O-$D_c$-$COR^{12}_c$ {où $D_c$ est alkylène ayant 1 à 4 atomes de carbone et $R^{12}_c$ est tel que défini ci-dessus}, ou bien $NHCOR^{13}$ {où $R^{13}_c$ est hydrogène, alkyle ayant 1 à 6 atomes de carbone, alcoxy ayant 1 à 6 atomes de carbone, $COR^{14}_c$ (où $R^{14}$ est hydroxy ou alcoxy ayant 1 à 6 atomes de carbone) ou $NHR^{15}_c$ (où $R^{15}_c$ est hydrogène ou alkyle ayant 1 à 6 atomes de carbone)}, ou phénoxy {où le noyau benzène peut être substitué par chacun des autres substituants $W_c$ ou $X_c$};

$Y_c$ et $Z_c$ peuvent être identiques ou différents et sont CH ou N;

$V_c$ est phényle, naphtyle, indényle, indanyle, 2-,3- ou 4-pyridyle, 2-,3- ou 5-pyrimidinyle, 2- ou 3-thiényle, 2- ou 3-furyle ou bien 2-ou 4- ou 5-thiazolyle, dont chacun peut être substitué par $W_c$ ou $X_c$ comme définis ci-dessus; et $R^9$ et $R^{10}$ sont indépendamment hydrogène ou alkyle ayant 1 à 6 atomes de carbone et des solvats de tels composés.

2. Utilisation selon la revendication 1 où, dans la formule $I_a$, $Y_a$ et $Z_a$ sont tous deux oxygène et/ou

ra est zéro et/ou

$R^5_a$ et $R^6_a$ sont tous deux hydrogène et/ou

qa est 2, 3 ou 4 et/ou

$R_a$ et $R'_a$ sont indépendamment hydrogène, méthyle, éthyle, propyle, isopropyle, n-butyle ou isobutyle et/ou

$A_a$ est phényle ou phényle substitué par un ou deux substituants $Q_a$ et/ou
$V_a$ est O, $S(O)_{na}$ ou N-$R^8{}_a$, de préférence 0.

3. Utilisation selon la revendication 1 ou 2 où dans la formule $I_a$ la totalité de a, b, c, et d sont CH ou a et d sont tous deux N et b et c sont tous deux CH ou d est N et a,b, et c sont CH.
4. Utilisation selon la revendication 1 où le composé de formule $I_a$ est
1′-phényl-spiro [cyclopentane-1,3′-(1-8)naphtyridine] -2′,4′-(1′H)-dione;
1′-phényl-spiro[cyclopentane-1,3′-quinoléine] -2′,4′-(1′H)-dione;
1′-(4-méthylphényl)spiro[cyclopentane-1,3′-(1,8)naphtyridine]-2′,4′-(1′H)-dione;
1′-(4-chlorophényl)spiro[cyclopentane-1,3′-(1,8)naphtyridine]-2′,4′-(1′H)-dione;
1′-(3,4-dichlorophényl)spiro[cyclopentane-1,3′-(1,8)naphtyridine]-2′,4′-(1′H)-dione;
1′-(3-chlorophényle)spiro[cyclopentane-1,3′-(1,8) naphtyridine] -2′,4′-(1′H)-dione
1′-(3-méthoxyphényl)spiro[cyclopentane-1,3′-(1,8)naphtyridine]-2′,4′-(1′H)-dione;
1′-(3-hydroxyphényl)spiro[cyclopentane-1,3′-(1,8)naphtyridine]-2′,4′-(1′H)-dione;
1′-phénylspiro[cyclopentane-1,3′-quinoléine] -2′,4′-(1′H)-dione;
1-phényl-3′,4′,5′,6′-tétrahydrospiro-[1,8-naphtyridine-3,2′-(2H)pyran]-2,4-dione;
1-(3-méthoxyphényl)-3′,4′,5′,6′-tétrahydrospiro-[1,8-naphtyridine-3,2′-(2H)pyran]-2,4-dione;
4,5-dihydro-1′-phényl-spiro[furan-2(3H),3′(2′H)-(1,8)naphtyridine]-2′,4′(1′H)-dione;
1-phényl-spiro[1,8-naphtyridine-3,2′-oxétane]-2,4-dione; ou
1-(3-chlorophényl)-3′,4′,5′, 6′-tétrahydrospiro-[1,8-naphtyridine-3,2′-(2H)pyran] -2,4-dione
leur solvat
5. Utilisation selon la revendication 1 où le composé est de la formule

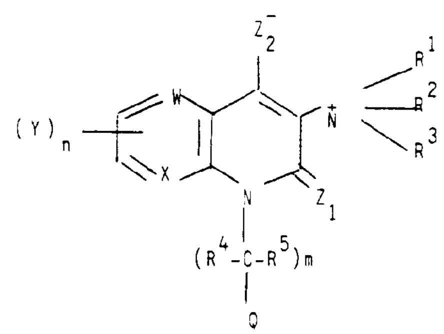

et ses sels, esters et solvats acceptables en pharmacie, dans laquelle:

W et X peuvent être identiques ou différents et chacun représente indépendamment CH ou N;

$Z_1$ et $Z_2$ sont identiques ou différents et chacun représente indépendamment O ou S;

$R^1$,$R^2$,$R^3$,$R^4$ et $R^5$ sont identiques ou différents et chacun peut être indépendamment choisi dans le groupe consistant en H, alkyle ayant 1 à 12 atomes de carbone, alcényle ayant 3 à 8 atomes de carbone, alkynyle ayant 3 à 8 atomes de carbone, alcoxyalkyle ayant 1 à 6 atomes de carbone dans la portion alcoxy et 2 à 6 atomes dans la portion alkyle, hydroxyalkyle ayant 2 à 8 atomes de carbone, cycloalkyle ayant 3 à 8 atomes de carbone, acyloxyalkyle ayant 1 à 6 atomes de carbone dans la portion acyloxy et 2 à 8 atomes de carbone dans la portion alkyle et -$R^6$-$CO_2R^0$ où $R^6$ représente un groupe alkyle ayant 1 à 6 atomes de carbone et $R^0$ représente hydrogène où un groupe alkyle ayant 1 à 6 atomes de carbone à condition que dans $R^1$, $R^2$ et $R^3$, OH du groupe hydroxyalkyle et l'acyloxy du groupe acyloxyalkyle ne soient pas joints à un atome de carbone qui est attaché à l'atome d'azote auquel $R^1$, $R^2$ et $R^3$ sont attachés et que, quand $R^1$, $R^2$ et/ou $R^3$ sont alcényle ou alkynyle, il y ait au moins une simple liaison carbone-carbone entre l'atome d'azote auquel $R^1$,$R^2$ et $R^3$ sont attachés et la double ou triple liaison carbone-carbone;

de plus, l'un de $R^1$, $R^2$ ou $R^3$ peut être phényle qui peut être substitué par un à trois substituants Y comme définis ci-dessous;

de plus encore, deux de $R^1$, $R^2$ et $R^3$ peuvent être joints pour représenter un noyau qui peut contenir 2 à 8 atomes de carbone, ledit noyau contenant facultativement un groupe-O-, -S- et/ou -$NR^4$- dans le noyau (où $R^4$ est tel que défini ci-dessus) et/ou contenant facultativement une double liaison carbone-carbone et ledit noyau étant facultativement substitué par un à trois substituants $R^7$ additionnels, lesquels substituants peuvent être identiques ou différents et chacun est indépendamment choisi parmi OH à condition que OH ne soit pas sur un carbone déjà joint à un hétéroatome, acyloxy ayant 1 à 6 atomes de carbone, hydroxyalkyle ayant 1 à 8 atomes de carbone, alcoxyalkyle ayant 1 à 6 atomes de carbone dans chacune de ses portions alcoxy et

alkyle, alkyle ayant 1 à 6 atomes de carbone, alcényle ayant 3 à 8 atomes de carbone, alkynyle ayant 3 à 8 atomes de carbone ou bien deux groupes substituants $R^7$ peuvent représenter un noyau hydrocarbure ayant 4 à 8 atomes de carbone au total:

de plus encore, les trois $R^1$, $R^2$ et $R^3$ avec l'atone d'azote auquel ils sont attachés peuvent être joints pour représenter un noyau de quinuclidine ou d'aza-bicyclo-[3.2.2] nonane, lequel noyau peut facultativement être substitué par un à trois groupes substituants $R^7$ tels que définis ci-dessus;

m est un nombre entier de 0 à 3;

n est un nombre entier de 0 à 2;

Q représente thiazolyle ou phényle facultativement substitué par un à trois substituants Y tels que définis ci-dessous;

chaque substituant Y est indépendamment choisi dans le groupe consistant en hydroxy, alkyle ayant 1 à 6 atomes de carbone, halogène, $NO_2$, alcoxy ayant 1 à 6 atomes de carbone, trifluorométhyle, cyano, cycloalkyle ayant 3 à 7 atomes de carbone, alcényloxy ayant 3 à 6 atomes de carbone, alkynyloxy ayant 3 à 6 atomes de carbone, hydroxyalkyle ayant 1 à 6 atomes de carbone, $-S(O)_n-R^8$ (où $R^8$ représente alkyle ayant 1 à 6 atomes de carbone et n est tel que défini ci-dessus), $-SO_2NH_2$, $-CO-R^9$ (où $R^9$ représente OH, $-NH-R^8$ ou $-O-R^8$, où $R^8$ est tel que défini ci-dessus), $-O-B-COR^9$ (où B représente un groupe alkylène ayant 1 à 4 atomes de carbone et $R^9$ est tel que défini ci-dessus), $-NH_2$, -NHCHO, $-NH-CO-R^9$ (où $R^9$ est tel que défini ci-dessus, à condition que ce ne soit pas hydroxy), $-NH-COCF_3$, $-NH-SO_2R^8$ (où $R^8$ est tel que défini ci-dessus), et $-NHSO_2CF_3$; et

3-diméthylamino-4-hydroxy-1-phényl-2-quinolone et 1-benzyl-3-diméthylamino-4-hydroxy-1-phényl-2-quinolone sont exclus.

6. Utilisation selon la revendication 5, où, dans la formule, au moins l'un de W et X est -N=.

7. Utilisation selon la revendiration 6 où, dans la formule, X est -N=, W est -CH= et $Z_1$ et $Z_2$ sont tous deux 0.

8. Utilisation selon l'une quelconque des revendications 5 à 7 où, dans la formule, n et m sont zéro.

9. Utilisation selon la revendication 5 où le composé est choisi parmi:

hydroxyde de 1-(1,2-dihydro-4-hydroxy-1-phényl-2-oxo-1,8 naphtyridin-3-yl)-1-méthyl-pyrrolidinium, sel interne;

hydroxyde de 1-(1,2-dihydro-4-hydroxy-1-phényl-2-oxo-1,8 -naphtyridin-3-yl)-pyrrolidinium, sel interne;

hydroxyde de 1-(1,2-dihydro-4-hydroxy-1-phényl-2-oxo-1,8 -naphtyridin- 3-yl)-4-hydroxy-pipéridinium, sel interne;

hydroxyde de 1-(1,2-dihydro-4-hydroxy-1-phényl-2-oxo-1,8 -naphtyridin- 3-yl)-4-hydroxy-pipéridinium, sel interne:

hydroxyde de 1-(1,2-dihydro-4-hydroxy-1-phényl-2-oxo-1,8 -naphtyridin- 3-yl)-quinuclidinium, sel interne;

hydroxyde de 1-(1,2-dihydro-4-hydroxy-1-phényl-2-oxo-1,8 -napthyridin- 3-yl)-1-méthyl-morpholinium, sel interne;

hydroxyde de 1-(1,2-dihydro-4-hydroxy-1-phényl-2-oxo-1,8 -naphtyridin-3-yl)-pipéridinium, sel interne; ou

hydroxyde de 1-(1,2-dihydro-4-hydroxy-1-phényl-2-oxo-1,8 -naphtyridin-3-yl)-2-hydroxyméthyl-pipéridinium, sel interne;

ou leurs sels, esters et solvats acceptables en pharmacie.

10. Utilisation selon la revendication 9 où le composé est l'hydroxyde de 1-(1,2-dihydro-4-hydroxy-1-phényl-2-oxo-1,8-napthyridin-3-yl)-1-méthyl-pyrrolidinium, sel interne, et ses sels, esters ou solvats acceptables en pharmacie.

11. Utilisation selon la revendication 1 où, dans la formule $I_c$, nc est zéro et/ou.

$Y_c$ est CH.

12. Utilisation selon la revendication 1 où le composé de formule $I_c$ a la formule de structure:

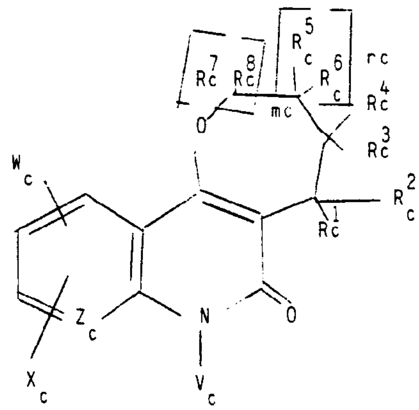

dans laquelle $W_c$, $X_c$, $Z_c$, $V_c$, $R^1{}_c$ à $R^8{}_c$, rc et mc sont tels que définis dans la revendication 1 dans laquelle, de préférence,

$Z_c$ est N et/ou
rc et mc sont tous deux zéro ou 1 ou
la somme de rc et mc est 1 et/ou
$B_c$ est oxygène et/ou
$V_c$ est

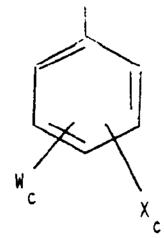

13. Utilisation selon la revendication 1 où le composé de formule $I_c$ a la formule de structure:

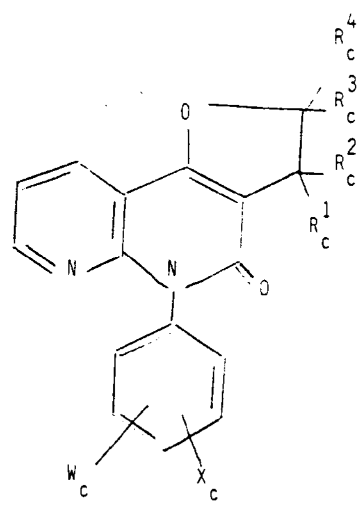

dans laquelle $W_c$, $X_c$, $R^1{}_c$, $R^2{}_c$, $R^3{}_c$, $R^4{}_c$ sont tels que définis dans la revendication 1, de préférence $R^1{}_c$, $R^2{}_c$, $R^3{}_c$ et $R^4{}_c$ sont hydrogène ou méthyle.

14. Utilisation selon la revendication 1 où ledit composé de formule $I_c$ a la formule de structure:

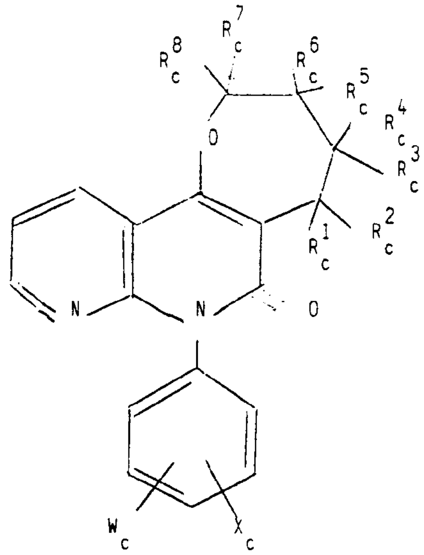

dans laquelle $W_c$,$X_c$ et $R^1_c$ -$R^8_c$ sont tels que définis dans la revendication 1, de préférence, $R^1_c$ -$R^8_c$ sont hydrogène ou méthyle, en particulier hydrogène,
ou l'un de $R^1_c$ -$R^8_c$ est méthyle et le reste est hydrogène et/ou
$W_c$ est 3-chloro and $X_c$ est hydrogène, chlore ou fluor ou
$W_c$ est 3-méthoxy and $X_c$ est hydrogène ou fluor ou
$W_c$ et $X_c$ sont tous deux hydrogène.

15. Utilisation selon la revendication 1 où ledit composé de formule $I_c$ a la formule de structure:

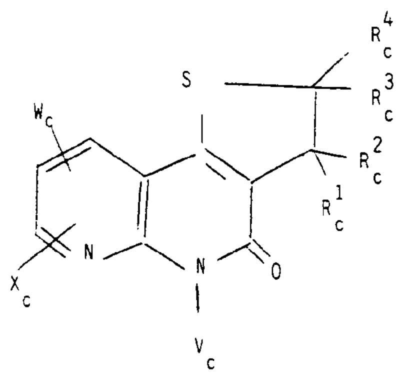

dans laquelle $W_c$, $X_c$, $V_c$, $R^1_c$ , $R^2_c$ , $R^3_c$ et $R^4_c$ sont tels que définis dans la revendication 1.

16. Utilisation selon la revendication 1 où ledit composé a la formule de structure:

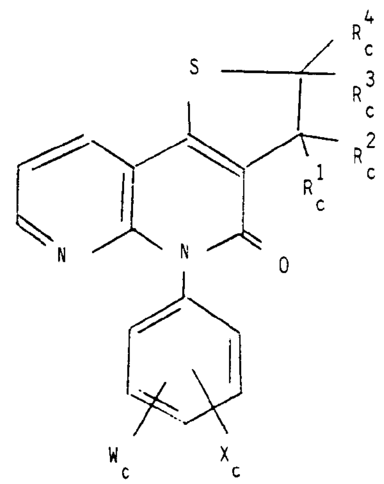

dans laquelle $W_c$, $X_c$, $R^1_c$, $R^2_c$, $R^3_c$, et $R^4_c$ sont tels que définis dans la revendication 1, de préférence
$W_c$ est 3-chloro et $X_c$ est hydrogène, chlore ou fluor ou
$W_c$ est 3-méthoxy et $X_c$ est hydrogène ou fluor ou
$W_c$ et $X_c$ sont tous deux hydrogène

17. Utilisation selon la revendication 1 où le composé de formule $I_c$ est:
3,5-dihydro-5-phényl-furo-[3,2-c][1,8] naphtyridin-4-[2H] -one;
3,5-dihydro-5-phényl-thiéno[3,2-c][1,8]-naphtyridin-4-[2H] -one;
6-phényl-2,3,4,6-tetrahydro-pyrano[3,2-c][1,8] -naphtyridin-5-one;
2-méthyl-3,5-dihydro-5-phényl-furo [3,2-c][1,8] -naphtyridin-4[2H] -one;
3,9-dihydro-9-phényl-furo[2,3-b][1,8] naphtyridin-4[2H]-one;
3,9-dihydro-9-(p-méthylphényl)-furo [2,3-b][1,8] -naphtyridin-4[2H]-one;
3,9-dihydro-2-méthyl-9-phényl-furo [2,3-b][1,8]-naphtyridin-4[2H]-one;
3,5-dihydro-5-(p-méthylphényl)-furo [3,2-c][1,8]-naphtyridin-4[2H]-one;
3,5-dihydro-5-(p-fluorophényl)-furo[3,2-c][1,8]-naphtyridin-4[2H]-one;
3,5-dihydro-5-(m-méthoxyphényl)-furo[3,2-c][1,8]-naphtyridin-4[2H]-one;
3,5-dihydro-5-(m-méthylthiophényl)-furo[3,2-c][1,8] -naphtyridin-4 [2H]-one;
3,9-dihydro-9-(p-fluorophényl)-furo[2,3-b][1,8]-naphtyridin-4[2H]-one;
3,9-dihydro-9-(m-méthoxyphényl)-furo[2,3-b][1,8]-naphtyridin-4[2H]-one;
3,9-dihydro-9-(m-méthylthiophényl)-furo[3,2-c][1,8]-naphtyridin-4[2H]-one;3,5-dihydro-5-(3,4-dichlorophényl)-furo-[3,2-c][1,8]-naphtyridin-4[2H]-one;
3,5-dihydro-5-(3,4-dichlorophényl)-2-méthyl-furo[3,2-c]1,8-naphtyridin-4[2H]-one;
3,5-dihydro-5(4-chlorophényl)-furo[3,2-c][1,8] -naphtyridin-4[2H]-one;
3,5-dihydro-5-(3-chlorophényl)-furo[3,2-c]1,8 -naphtyridin-4[2H]-one;
3,5-dihydro-5-(3-chlorophényl)-2-méthyl-furo[3,2-c]-[1,8] -naphtyridin-4[2H]-one;
3,5-dihydro-5-(4-fluorophényl)-2-méthyl-furo[3,2-c]-[1,8]-naphtyridin-4[2H]-one;
3,5-dihydro-5-(3-méthoxyphényl)-2-méthyl-furo-[3,2-c]-[1,8]-naphtyridin-4[2H] -one;
3,5-dihydro-5-(3,5-dichlorophényl)-furo[3,2-c]-[1,8]-naphtyridin-4[2H]-one;
3,5-dihydro-5-(3,5-dichlorophényl)-2-méthyl-furo[3,2-c]-[1,8]-naphtyridin-4[2H]-one;
3-5-dihydro-5-phényl-2,2-diméthyl-furo[3,2-c]-[1,8]-naphtyridin-4[2H]-one;
3,5-dihydro-5-(3-methylsulfonylaminophényl)-2-méthyl-furo[3,2-c][1,8]-naphtyridin-4[2H]-one;
3,5-dihydro-5(3-methylsulfonylaminophényl)-furo[3,2-c][1,8]-naphtyridin-4[2H]-one;
3,9-dihydro-9-(3,4-dichlorophényl)-furo[2,3-b][1,8]-naphtyridin-4[2H]-one;
3,9-dihydro-9-(4-chlorophényl)-furo[2,3-b][1,8]-naphtyridin-4[2H]-one;
3,9-dihydro-9-(3-chlorophényl)-furo[2,3-b][1,8]-naphtyridin-4[2H]-one;
3,9-dihydro-9-(3-chlorophényl)-2-méthyl-furo[2,3-b]-[1,8]-naphtyridin-4[2H]-one;
3,9-dihydro-9-(4-fluorophényl)-2-méthyl-furo[2,3-b]-[1,8]-naphtyridin-4[2H]-one;
3,9-dihydro-9-(3-méthoxyphényl)-2-méthyl-furo[2,3-b]-[1,8]-naphtyridin-4[2H]-one;
3,9-dihydro-9-(3,5-dichlorophényl)-furo[2,3-b][1,8]-naphtyridin-4[2H]-one;
3,9-dihydro-9-(3,5-dichlorophényl)-2-méthyl-furo[2,3-b]-[1,8]-naphtyridin-4[2H]-one;
3,9-dihydro-9-(3-méthylsulfonylaminophényl)-2-méthyl-furo[2,3-b][1,8]-naphtyridin-4[2H]-one;
6-(4-chlorophényl)-2,3,4,6-tétrahydro-5H-pyrano[3,2-c][1,8]naphtyridin-5-one;

6-(3,4-dichlorophényl)-2,3,4,6-tétrahydro-5H-pyrano-[3,2-c][1,8]naphtyridin-5-one;
6-(4-méthoxyphényl)-2,3,4,6-tétrahydro-5H-pyrano[3,2-c][1,8]naphtyridin-5-one;
6-(4-méthylphényl)-2,3,4,6-tétrahydro-5H-pyrano[3,2-c][1,8]naphtyridin-5-one;
10-(3,4-dichlorophényl)-2,3,4,10-tétrahydro-5-H-pyrano-[2,3-b][1,8]naphtyridin-5-one;
10-(4-méthoxyphényl)-2,3,4,10-tétrahydro-5H-pyrano -[2,3-b][1,8]naphtyridin-5-one;
10-(4-chlorophényl)-2,3,4,10-tétrahydro-5H-pyrano-[2,3-b][1,8]naphtyridin-5-one;
10-(4-méthylphényl)-2,3,4,10-tétrahydro-5H-pyrano-[2,3-b][1,8]naphtyridin-5-one;
10-phényl-2,3,4,10-tétrahydro-5H-pyrano-[2,3-b][1,8]-naphtyridin-5-one;
7-phényl-3,4,5,7-tétrahydro-oxépino[3,2-c][1,8]-naphtyridin-6[2H]-one;
7-(4-chlorophényl)-3,4,5,7-tétrahydro-oxépino-[3,2-c][1,8]-naphtyridin-6[2H]-one;
7-(3-chlorophényl)-3,4,5,7-tétrahydro-oxépino-[3,2-c][1,8]-naphtyridin-6[2H]-one;
7-(3-méthoxyphényl)-3,4,5,7-tétrahydro-oxépino-[3,2-c][1,8]-naphtyridin-6[2H]-one; ou
7-(3-hydroxyphényl)-3,4,5,7-tétrahydro-oxépino-[3,2-c][1,8]-naphtyridin-6[2H]-one.